(19) Europäisches Patentamt
European Patent Office
Office européen des brevets

(11) EP 4 353 757 A1

(12) EUROPEAN PATENT APPLICATION
published in accordance with Art. 153(4) EPC

(43) Date of publication:
**17.04.2024 Bulletin 2024/16**

(21) Application number: **22858787.9**

(22) Date of filing: **19.08.2022**

(51) International Patent Classification (IPC):
***C08F 220/06*** $^{(2006.01)}$ ***C08F 220/30*** $^{(2006.01)}$

(52) Cooperative Patent Classification (CPC):
**C08F 220/06; C08F 220/30**

(86) International application number:
**PCT/KR2022/012381**

(87) International publication number:
**WO 2023/022550 (23.02.2023 Gazette 2023/08)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.08.2021 KR 20210109663**
**16.08.2022 KR 20220102205**

(71) Applicant: **LG Chem, Ltd.**
**Seoul 07336 (KR)**

(72) Inventors:
- **KANG, Soonhee**
  **Daejeon 34122 (KR)**
- **CHO, Beomshin**
  **Daejeon 34122 (KR)**
- **HAM, Kyungrok**
  **Daejeon 34122 (KR)**
- **CHOI, Hyungsam**
  **Daejeon 34122 (KR)**
- **JUNG, Seonjung**
  **Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **SUPER ABSORBENT POLYMER AND PREPARATION METHOD THEREOF**

(57) Provided are a superabsorbent polymer and a method of preparing the same, more particularly, a superabsorbent polymer exhibiting an improved bacterial growth-inhibitory property without deterioration in absorption performances of the superabsorbent polymer, and a method of preparing the same.

【FIG. 1】

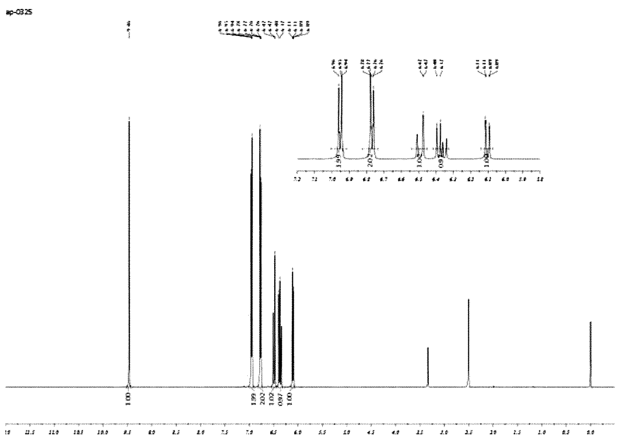

EP 4 353 757 A1

Processed by Luminess, 75001 PARIS (FR)

## Description

[Technical Field]

Cross-reference to Related Application

The present application is based on, and claims priority from, Korean Patent Application Nos. 10-2021-0109663, and 10-2022-0102205, filed on August 19, 2021, and August 16, 2022, respectively, the disclosures of which are hereby incorporated by reference herein in their entirety.

The present invention relates to a superabsorbent polymer exhibiting an improved bacterial growth-inhibitory property without deterioration in absorption performances of the superabsorbent polymer, and a method of preparing the same.

[Background Art]

A superabsorbent polymer (SAP) is a synthetic polymeric material capable of absorbing moisture from 500 to 1000 times its own weight. Various manufacturers have denominated it as different names, such as SAM (Super Absorbency Material), AGM (Absorbent Gel Material), etc. Since such superabsorbent polymers started to be practically applied in sanitary products, now they have been widely used not only for hygiene products such as paper diapers for children, etc., but also for water retaining soil products for gardening, water stop materials for the civil engineering and construction, sheets for raising seedling, fresh-keeping agents for food distribution fields, materials for poultice, electrical insulation applications.

In particular, superabsorbent polymers are most widely applied to hygiene products such as paper diapers for children, diapers for adults, or disposable absorbent products. Therefore, when bacteria grow in these hygiene products and disposable absorbent products, there is a problem in that secondary odors as well as various diseases are induced. Accordingly, attempts have been made to introduce various bacterial growth inhibitory components, or deodorizing or antibacterial functional components into superabsorbent polymers.

However, in the attempts to introduce antibacterial agents capable of inhibiting bacterial growth into superabsorbent polymers, it is not easy to select and introduce an antibacterial agent component that exhibits excellent bacterial growth-inhibiting and deodorizing properties, is harmless to the human body, and satisfies economic feasibility without deteriorating basic physical properties of the superabsorbent polymers.

Accordingly, there is a continuous demand for the development of a superabsorbent polymer-related technology capable of remarkably inhibiting bacterial growth without deteriorating basic physical properties of superabsorbent polymers.

[Disclosure]

[Technical Problem]

Accordingly, there are provided a superabsorbent polymer capable of exhibiting an improved bacterial growth-inhibiting property without deterioration in absorption performances of the superabsorbent polymer, and a method of preparing the same.

[Technical Solution]

According to one embodiment of the present invention,
there is provided a superabsorbent polymer including a crosslinked polymer of an acrylic acid-based monomer including acidic groups of which at least part is neutralized; a polymerizable antibacterial monomer represented by the following Formula 1; and a crosslinking agent:

[Formula 1]

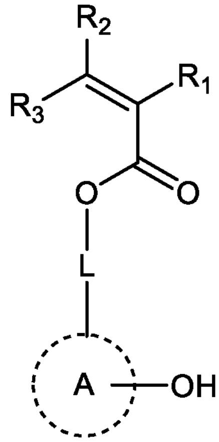

in Formula 1,

$R_1$ to $R_3$ are each independently hydrogen, alkyl having 1 to 4 carbon atoms, or a carboxyl group (COOH),
L is a single bond; or arylene having 6 to 60 carbon atoms, and
A is an aromatic ring having 6 to 60 carbon atoms,
wherein the arylene and the aromatic ring are each independently unsubstituted or substituted with one or more substituents selected from the group consisting of hydroxy, alkyl having 1 to 10 carbon atoms, and alkoxy having 1 to 10 carbon atoms.

According to another embodiment of the present invention, there is provided a method of preparing a superabsorbent polymer, the method including the steps of:

preparing a monomer composition by mixing an acrylic acid-based monomer including acidic groups, a polymerizable antibacterial monomer represented by Formula 1, a crosslinking agent, and a polymerization initiator (step 1);
forming a water-containing gel polymer by polymerizing the monomer composition (step 2); and
drying, pulverizing, and classifying the water-containing gel polymer (step 3).

Furthermore, according to still another embodiment of the present invention, there is provided an article including the above-described superabsorbent polymer.

[Effect of the Invention]

A superabsorbent polymer of the present invention may exhibit an antibacterial property of inhibiting bacterial growth which may be harmful to the human body and may cause secondary odors.

Specifically, since the superabsorbent polymer is prepared by using a polymerizable antibacterial monomer having a specific structure during formation of a crosslinked polymer, it may exhibit an antibacterial property against at least one of Gram-positive bacteria and Gram-negative bacteria while maintaining excellent water retention capacity, unlike those prepared by using another antibacterial agent.

Further, since the polymerizable antibacterial monomer is copolymerized with an acrylic acid monomer, the risk of leakage of the polymerizable antibacterial monomer is reduced, as compared with the case where the polymerizable antibacterial monomer or a polymer of the polymerizable antibacterial monomer is simply mixed in the superabsorbent polymer, and thus there may be no risk of harming human body due to leakage of the antibacterial agent.

Accordingly, the superabsorbent polymer may be very preferably applied to various hygiene products, such as diapers for children as well as diapers for adults requiring an antibacterial property against bacteria, etc.

[Brief Description of Drawings]

FIG. 1 shows an $^1H$ NMR spectrum of a polymerizable antibacterial monomer 1-1.

[Best Mode for Carrying Out the Invention]

The terms used in this description are just for explaining exemplary embodiments and it is not intended to restrict the present invention. The singular expression may include the plural expression unless it is differently expressed contextually. It must be understood that the term "include", "equip", or "have" in the present description is only used for designating the existence of characteristics taken effect, steps, components, or combinations thereof, and do not exclude the existence or the possibility of addition of one or more different characteristics, steps, components or combinations thereof beforehand.

Further, in the present invention, when a layer or an element is mentioned to be formed "on" or "above" layers or elements, it means that each layer or element is directly formed on the layers or elements, or other layers or elements may be formed between the layers, subjects, or substrates.

The present invention may be variously modified and have various forms, and specific exemplary embodiments are exemplified and explained in detail in the following description. However, it is not intended to limit the present invention to the specific exemplary embodiments and it must be understood that the present invention includes every modifications, equivalents, or replacements included in the spirit and technical scope of the present invention.

Further, the technical terms used in this description are just for mentioning specific exemplary embodiments and it is not intended to restrict the present invention. The singular expression used herein may include the plural expression unless it is differently expressed contextually.

Meanwhile, as used herein, the term "(meth)acrylate" includes both acrylates and methacrylates.

Further, as used herein, the alkyl group may be linear or branched, and its number of carbon atoms is, but not particularly limited to, preferably 1 to 20. According to one embodiment, the number of carbon atoms of the alkyl group is 1 to 10. According to another embodiment, the number of carbon atoms of the alkyl group is 1 to 6. Specific examples of the alkyl group may include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, 1-ethyl-propyl, 1,1-dimethylpropyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, 2-methylhexyl, 3-methylhexyl, 4-methylhexyl, 5-methylhexyl, octyl, n-octyl, tert-octyl, 1-methylheptyl, 2-ethylhexyl, 2,4,4-trimethyl-1-pentyl, 2,4,4-trimethyl-2-pentyl, 2-propylpentyl, n-nonyl, 2,2-dimethylheptyl, etc., but are not limited thereto. In the present specification, the above-described description of the alkyl group may be applied to alkylene, except that alkylene is a divalent group.

Further, as used herein, the alkoxy group may be linear or branched, and its number of carbon atoms is, but not particularly limited to, preferably 1 to 10. According to one embodiment, the number of carbon atoms of the alkoxy group is 1 to 6. Specific examples of the alkoxy group may include methoxy, ethoxy, n-propoxy, isopropoxy, n-butoxy, etc., but are not limited thereto.

Further, as used herein, the aryl group is not particularly limited, but preferably has 6 to 60 carbon atoms, and it may be a monocyclic aryl group or a polycyclic aryl group. According to one embodiment, the number of carbon atoms of the aryl group is 6 to 30. According to one embodiment, the number of carbon atoms of the aryl group is 6 to 20. The aryl group may be a phenyl group, a biphenyl group, a terphenyl group, etc., as the monocyclic aryl group, but is not limited thereto. The polycyclic aryl group may include a naphthyl group, an anthryl group, a phenanthryl group, a pyrenyl group, a perylenyl group, a chrysenyl group, a fluorenyl group, etc., but is not limited thereto. Further, in the present specification, the above-described description of the aryl group may be applied to arylene, except that arylene is a divalent group.

Further, as used herein, the aromatic ring refers to a monocyclic or condensed polycyclic ring containing only carbon as a ring-forming atom and having aromaticity in the entire molecule. The number of carbon atoms of the aromatic ring is 6 to 60, or 6 to 30, or 6 to 20, but is not limited thereto. Further, the aromatic ring may be a benzene ring, a naphthalene ring, an anthracene ring, a phenanthrene ring, a pyrene ring, etc., but is not limited thereto.

As used herein, the term "polymer" refers to a polymerized state of acrylic acid-based monomers, and may encompass those of all water content ranges or particle size ranges. Among the polymers, those having a water content (a moisture content) of about 40% by weight or more after being polymerized and before being dried may be referred to as a water-containing gel polymer, and particles obtained by pulverizing and drying the water-containing gel polymer may be referred to as a crosslinked polymer.

Further, the term "superabsorbent polymer particle" refers to a particulate material including a crosslinked polymer which is obtained by polymerizing and crosslinking the acrylic acid-based monomer including acidic groups of which at least part is neutralized, by an internal crosslinking agent.

Further, the term "superabsorbent polymer" refers to, depending on the context, a crosslinked polymer obtained by polymerizing the acrylic acid-based monomer including acidic groups of which at least part is neutralized, or a base polymer in the form of powder, consisting of superabsorbent polymer particles obtained by pulverizing the crosslinked polymer, or is used to encompass those made suitable for commercialization through additional processes of the crosslinked polymer or the base polymer, for example, surface crosslinking, reassembling of fine particles, drying,

pulverizing, classifying, etc.

Traditionally, in order to secure antibacterial and deodorizing properties in the superabsorbent polymers, a metal compound having an antibacterial function or an organic compound containing a cation or alcohol functional group was introduced in the form of an additive. In this case, however, safety of the superabsorbent polymer was lowered, basic physical properties, such as absorption properties, etc., were lowered, and there were problems in persistence of antibacterial properties and leakage of antibacterial substances.

For example, an attempt has been made to introduce, into superabsorbent polymers, an antibacterial agent component containing an antibacterial metal ion such as silver, copper, copper, zinc, etc. This antibacterial metal ion-containing component may impart deodorizing properties by destroying the cell walls of microorganisms, such as bacteria, etc., and by killing bacteria with enzymes that may cause bad odor in the superabsorbent polymers. However, the metal ion-containing component is classified as a biocide material that may kill even microorganisms beneficial to the human body. As a result, when the superabsorbent polymers are applied to hygiene products such as diapers for children or adults, etc., introduction of the metal ion-containing antibacterial agent component is excluded as much as possible.

Traditionally, when the antibacterial agent inhibiting bacterial growth was introduced into a superabsorbent polymer, a method of mixing the superabsorbent polymer with a small amount of the antibacterial agent was mainly applied. However, when this mixing method is applied, it was difficult to uniformly maintain the bacterial growth-inhibiting property over time. Moreover, this mixing method may cause non-uniform application and separation of the antibacterial agent component during the process of mixing the superabsorbent polymer and the antibacterial agent, and there have been also disadvantages such as a need to install a new facility for the mixing.

Further, there are various types of bacteria such that more than 5,000 types of bacteria have been identified. Specifically, bacteria have a variety of cell morphologies such as a sphere shape, a rod shape, a spiral shape, etc., and the oxygen demand is also different between bacteria, and thus bacteria are divided into aerobic bacteria, facultative aerobic bacteria, and anaerobic bacteria. Therefore, it has not been easy for one type of antibacterial agent to have a physical/chemical mechanism by which cell membranes/cell walls of many different bacteria are damaged or proteins thereof are denatured.

However, it was found that when a superabsorbent polymer is prepared by polymerizing an acrylic acid-based monomer with a monomer containing a hydroxy group (OH) of a specific structure, it exhibits absorption performances above a predetermined level while exhibiting an antibacterial property against at least one of Gram-positive bacteria and Gram-negative bacteria, thereby completing the present invention.

Specifically, when the polymerizable antibacterial monomer represented by Formula 1 is in contact with bacteria, the aromatic ring structure substituted with a hydroxy group binds to a protein material essential for the metabolism of bacteria, thereby inhibiting bacterial growth. More specifically, the hydroxy group may reduce the bacterial metabolism through a reaction with a thiol group (sulfhydryl group, -SH) which may exist in the bacterial protein or through a non-binding interaction with other protein moieties, thereby inhibiting bacterial growth. Therefore, the superabsorbent polymer including the crosslinked polymer formed by the polymerizable antibacterial monomer represented by Formula 1 may exhibit antibacterial property against at least one of Gram-positive bacteria and Gram-negative bacteria.

Furthermore, since the superabsorbent polymer includes the antibacterial monomer in the form of the crosslinked polymer, in which the antibacterial monomer is crosslinked with the acrylic acid-based monomer, the antibacterial monomer does not remain in the form of a compound in the superabsorbent polymer. Accordingly, there is no concern about leakage of the antibacterial agent even over time, and thus the superabsorbent polymer is characterized by exhibiting excellent stability.

Hereinafter, a superabsorbent polymer and a preparation method thereof will be described in more detail according to specific embodiments of the present invention.

## Superabsorbent polymer

Specifically, a superabsorbent polymer according to one embodiment of the present invention is characterized by including a crosslinked polymer of an acrylic acid-based monomer including acidic groups of which at least part is neutralized; a polymerizable antibacterial monomer represented by the following Formula 1; and a crosslinking agent:

[Formula 1]

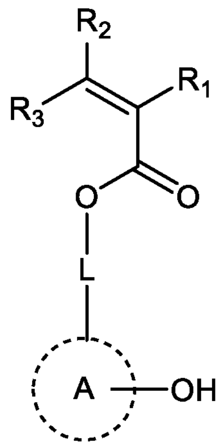

in Formula 1,

$R_1$ to $R_3$ are each independently hydrogen, alkyl having 1 to 4 carbon atoms, or a carboxyl group (COOH),
L is a single bond; or arylene having 6 to 60 carbon atoms, and
A is an aromatic ring having 6 to 60 carbon atoms,
wherein the arylene and the aromatic ring are each independently unsubstituted or substituted with one or more substituents, for example, 1 to 5 substituents selected from the group consisting of hydroxy, alkyl having 1 to 10 carbon atoms, and alkoxy having 1 to 10 carbon atoms.

In this regard, the crosslinked polymer, resulting from crosslinking polymerization of the acrylic acid-based monomer and the polymerizable antibacterial monomer in the presence of the crosslinking agent, has a three-dimensional network structure, in which the main chains formed by polymerization of the monomers are crosslinked by the crosslinking agent. Therefore, the polymerizable antibacterial monomer does not exist as a separate compound in the superabsorbent polymer, but exists as a repeating unit constituting the main chain, and thus it does not leak over time. Accordingly, the antibacterial property of the superabsorbent polymer may be continuously maintained.

Further, since the crosslinked polymer of the superabsorbent polymer further includes a repeating unit derived from the polymerizable antibacterial monomer, in addition to the repeating unit derived from the acrylic acid-based monomer, centrifuge retention capacity may be improved.

Meanwhile, in Formula 1, $R_1$ to $R_3$ may be each independently hydrogen, methyl, or a carboxyl group.

For example, $R_1$ to $R_3$ are all hydrogens; or one of $R_1$ to $R_3$ may be a methyl or carboxyl group, and the others may be hydrogens.

Further, in Formula 1, L may be a single bond; or may be arylene having 6 to 20 carbon atoms, which is unsubstituted or substituted with 1 to 3 substituents selected from the group consisting of hydroxy, alkyl having 1 to 10 carbon atoms, and alkoxy having 1 to 10 carbon atoms.

Specifically, L may be a single bond, phenylene, or naphthylene.

For example, L may be a single bond, or any one selected from the group consisting of the following compounds, but is not limited thereto:

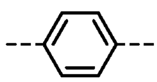 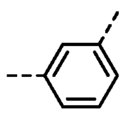 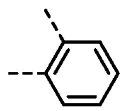

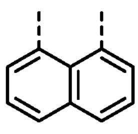 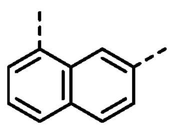 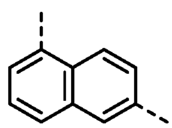 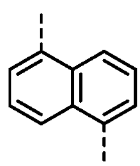 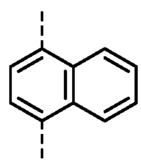

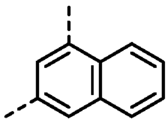
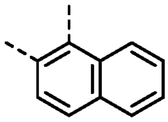
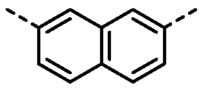
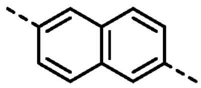
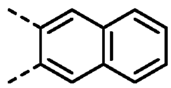

Further, in Formula 1, A may be an aromatic ring having 6 to 20 carbon atoms, which is unsubstituted or substituted with 1 to 3 substituents selected from the group consisting of hydroxy, alkyl having 1 to 10 carbon atoms, and alkoxy having 1 to 10 carbon atoms.

More specifically, A is a benzene or naphthalene ring,
wherein the benzene and naphthalene rings may be each independently unsubstituted or substituted with 1 to 3 substituents selected from the group consisting of hydroxy, alkyl having 1 to 10 carbon atoms, and alkoxy having 1 to 10 carbon atoms.

For example, A is a benzene or naphthalene ring,
wherein the benzene and naphthalene rings may be each independently unsubstituted or substituted with 1 to 3 substituents selected from the group consisting of hydroxy, methyl, ethyl, n-propyl, isopropyl, n-butyl, isobutyl, tert-butyl, sec-butyl, n-pentyl, n-hexyl, n-heptyl, n-octyl, 2,4,4-trimethyl-1-pentyl, 2,4,4-trimethyl-2-pentyl, methoxy, ethoxy, n-propoxy, and n-butoxy.

The polymerizable antibacterial monomer may be represented by any one of the following Formulae 1-1 to 1-3:

[Formula 1-1]

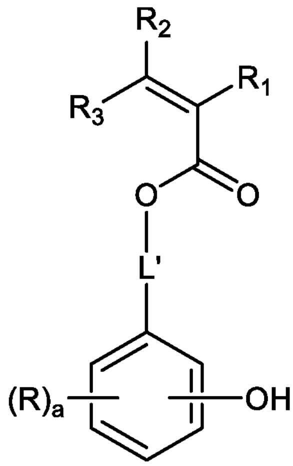

[Formula 1-2]

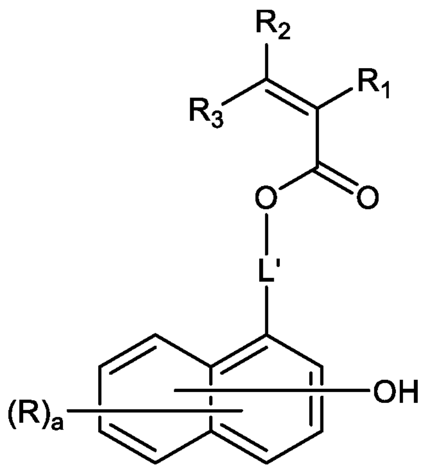

[Formula 1-3]

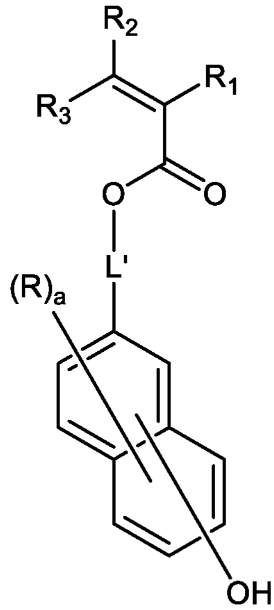

in Formulae 1-1 to 1-3,

L' is a single bond, phenylene, or naphthylene,
R is hydroxy, alkyl having 1 to 10 carbon atoms, or alkoxy having 1 to 10 carbon atoms,
a is an integer of 0 to 3,
when a is 2 or more, two or more R's are the same as or different from each other, and
$R_1$ to $R_3$ are as defined in Formula 1 above.

In Formulae 1-1 to 1-3, L may be a single bond, 1,4-phenylene, 1,3-phenylene, or 1,6-naphthylene, but is not limited thereto.

Further, in Formulae 1-1 to 1-3, a may be 0, 1, 2, or 3.

For example, the polymerizable antibacterial monomer may be any one selected from the group consisting of the following compounds, but is not limited thereto:

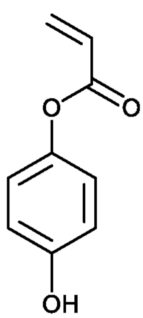

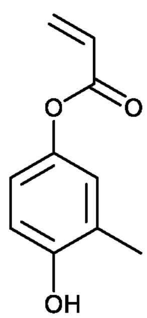

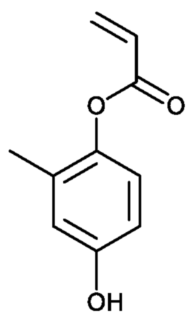

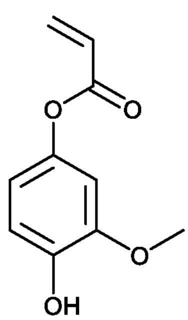

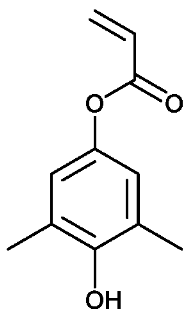
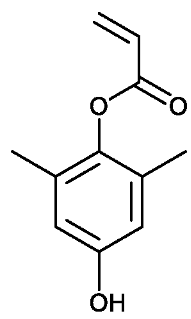
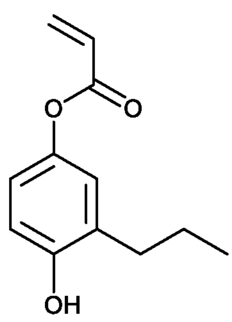

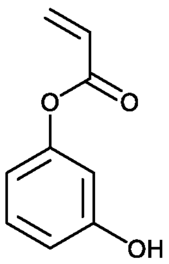
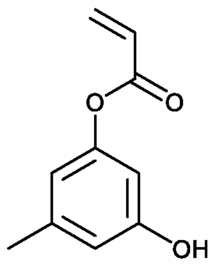
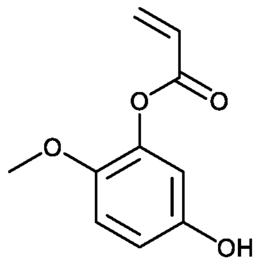
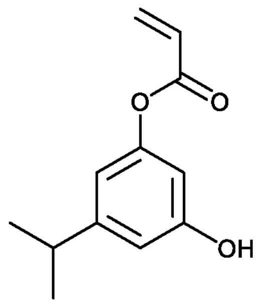

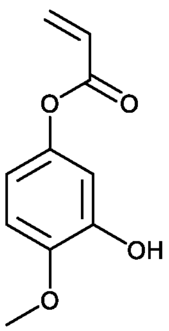
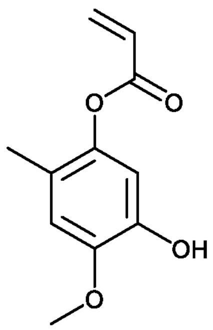
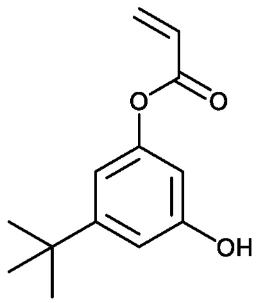
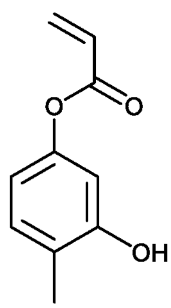

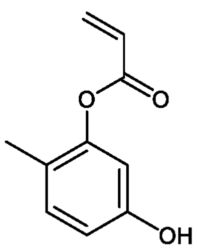
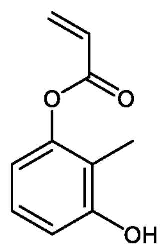
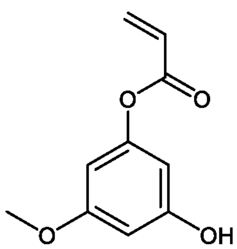
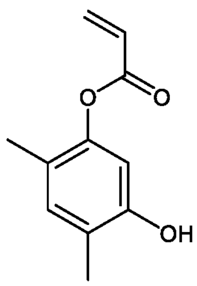

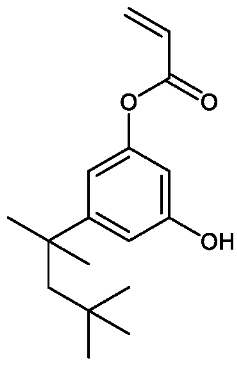

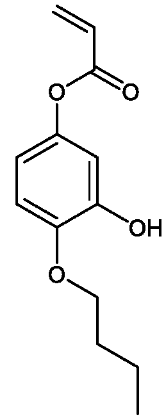

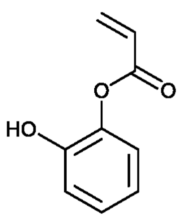

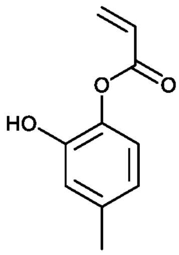

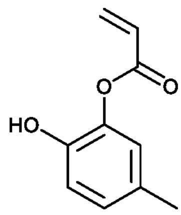

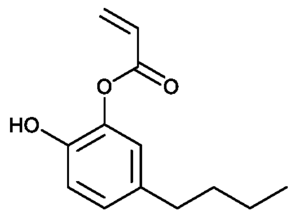

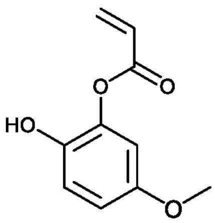

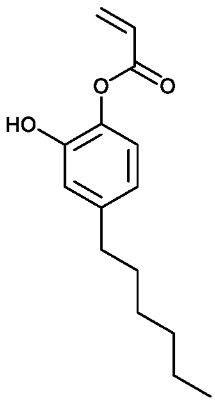

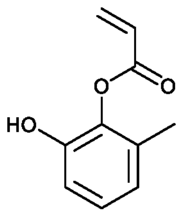

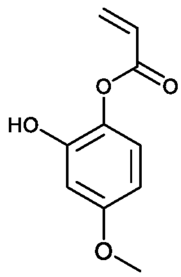

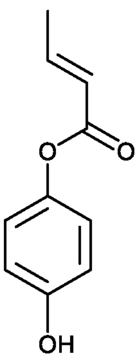

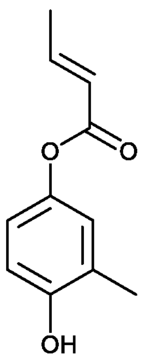

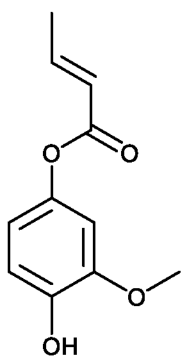

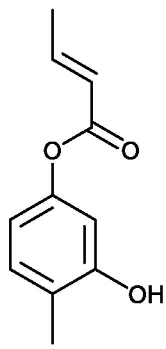

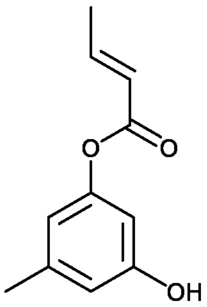
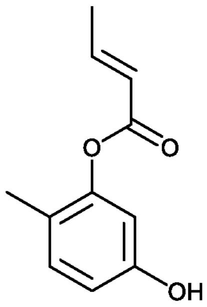
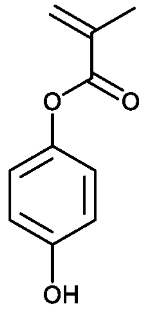
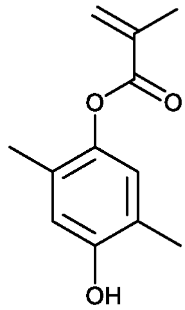
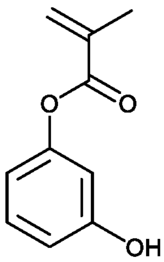
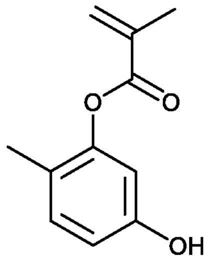
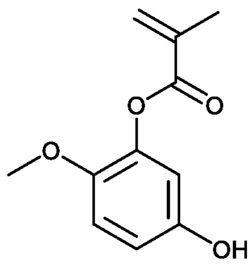
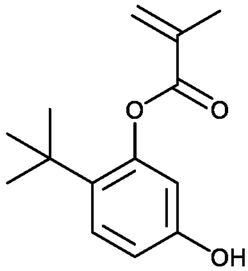
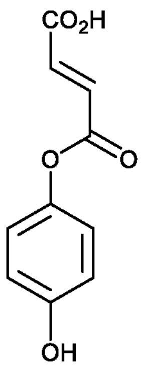
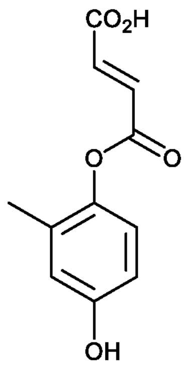
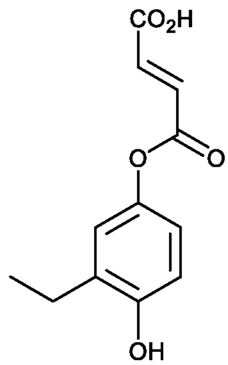
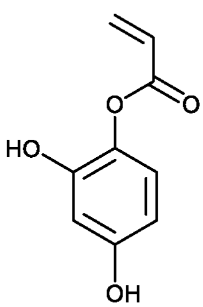
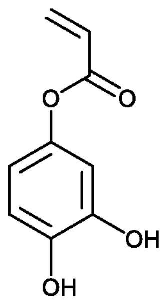
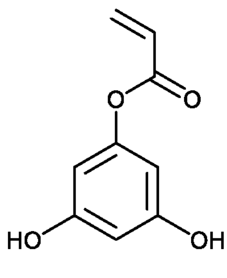
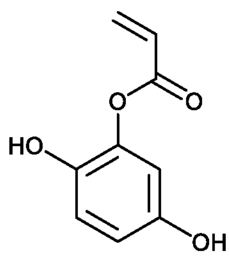
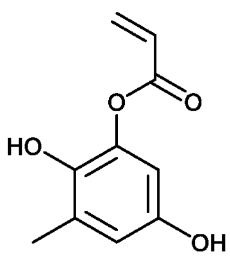

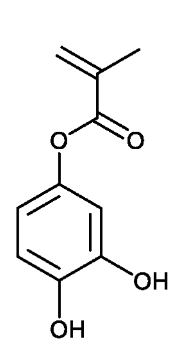
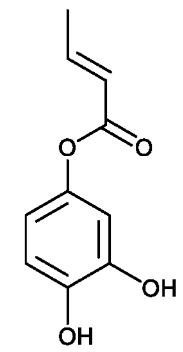
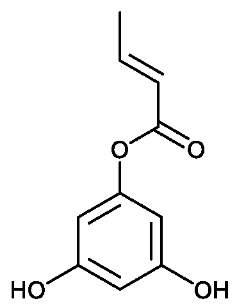
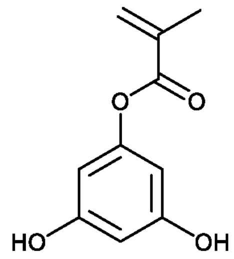

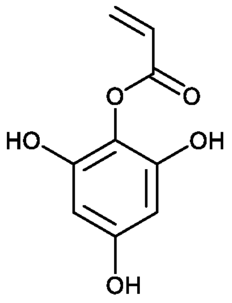
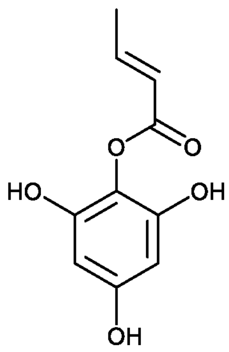
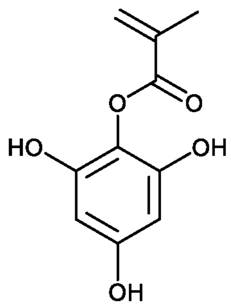
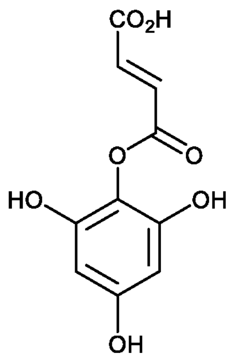

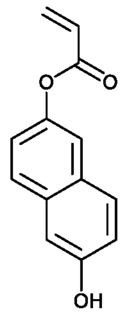
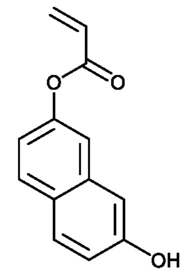
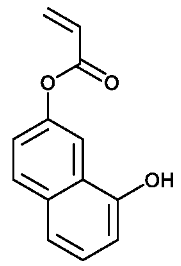
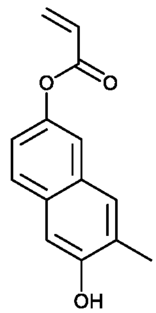

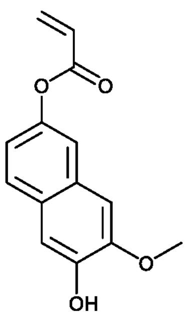
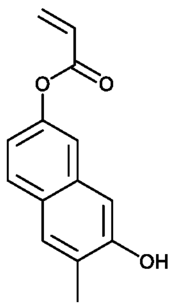
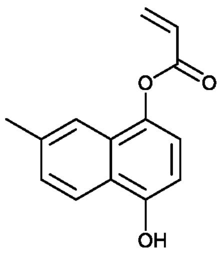
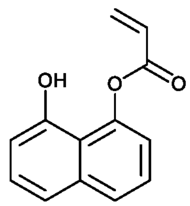

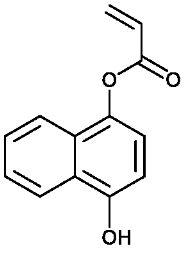
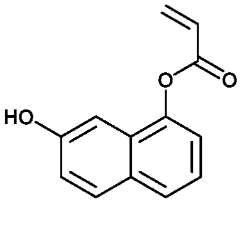
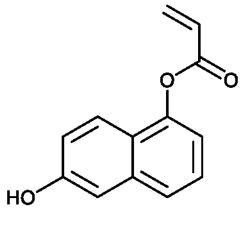
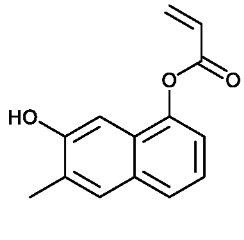

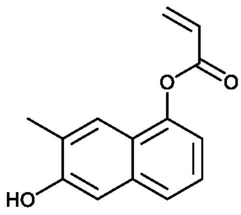
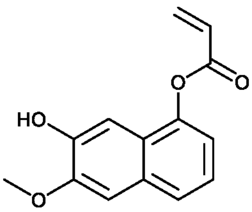
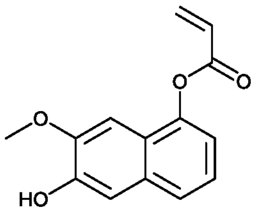
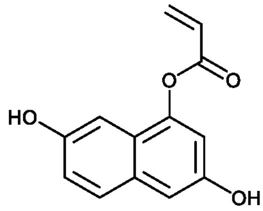

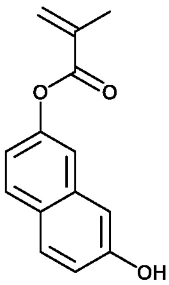
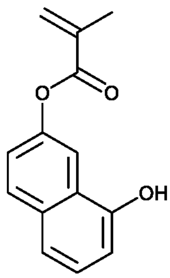
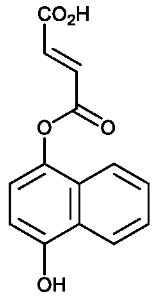
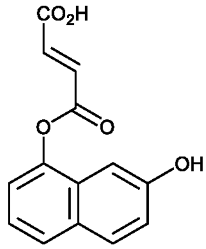

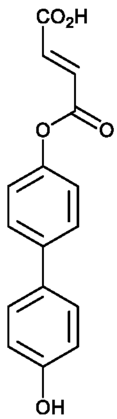
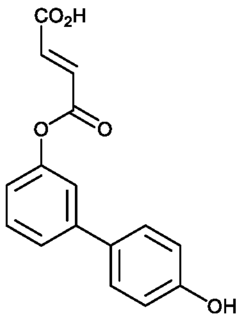

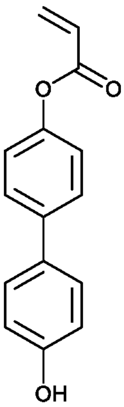
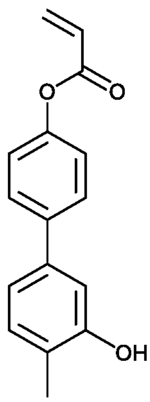
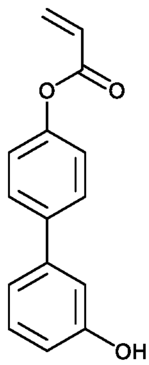
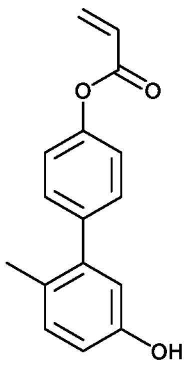

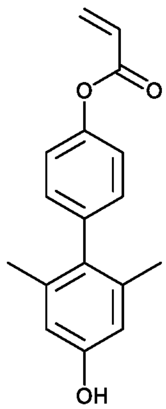

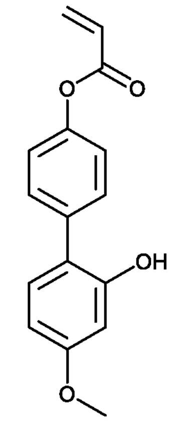

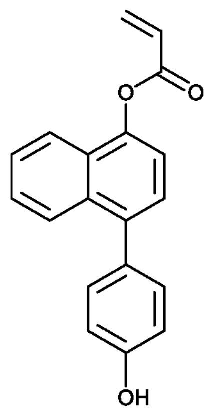

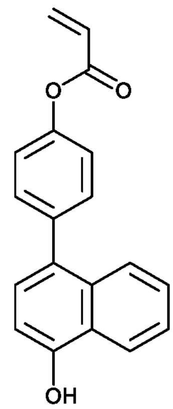

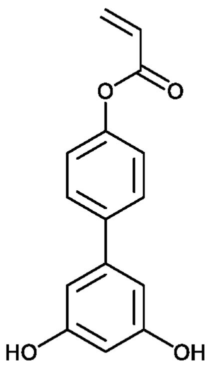

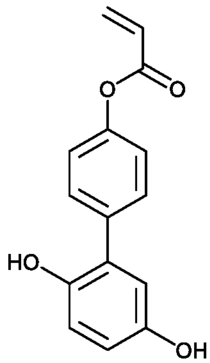

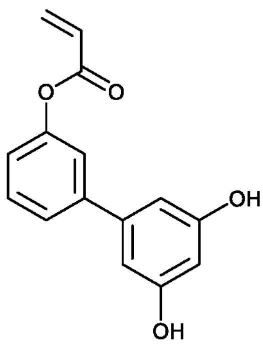

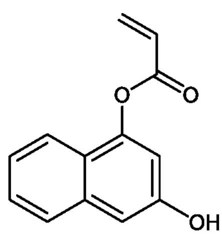

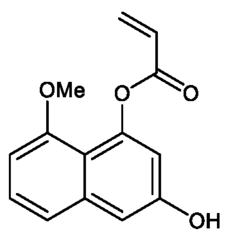

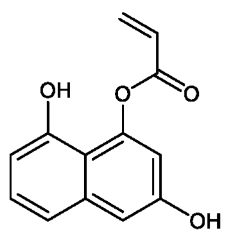

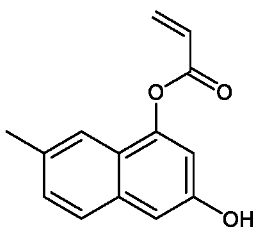

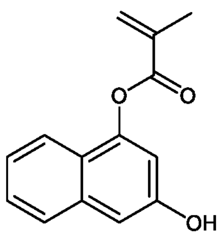

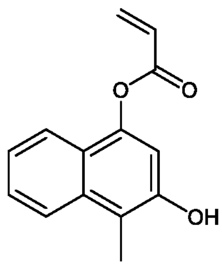

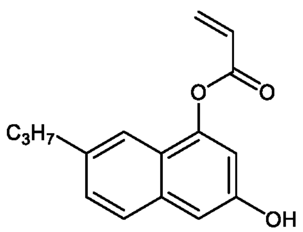

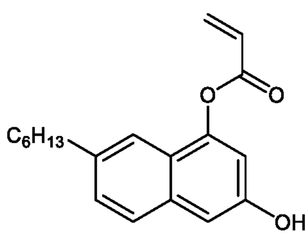

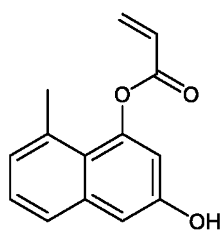

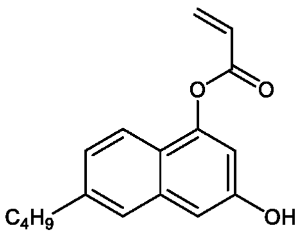

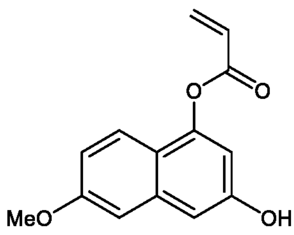

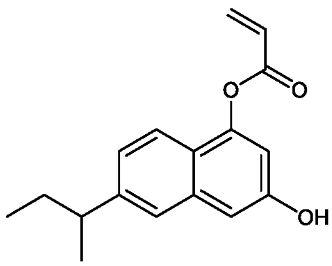

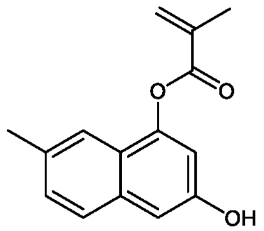

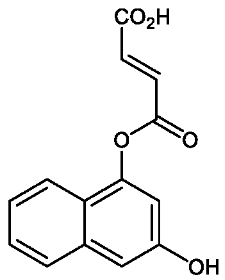

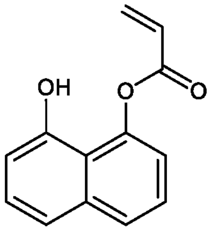

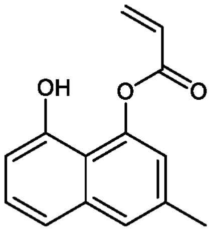

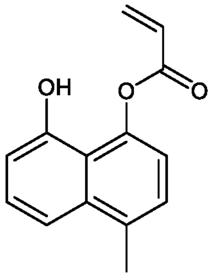

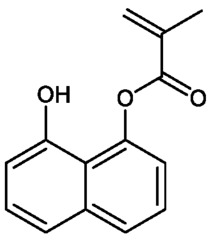

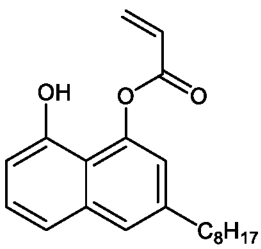

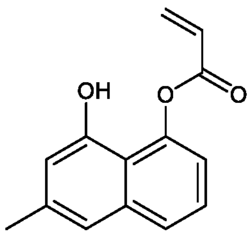

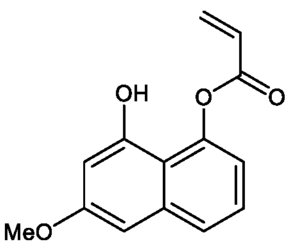

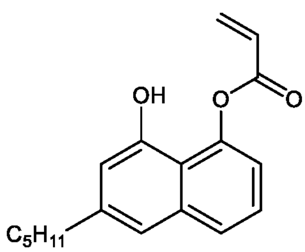

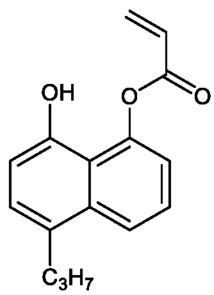

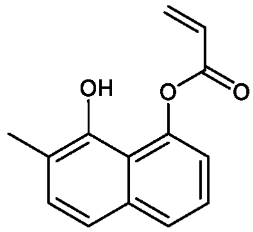

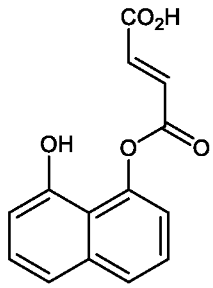

Furthermore, the polymerizable antibacterial monomer is included in the crosslinked polymer in an amount of 0.1 part by weight to 1 part by weight with respect to 100 parts by weight of the acrylic acid-based monomer. When the polymerizable antibacterial monomer is included in an amount of less than 0.1 part by weight with respect to 100 parts by weight of the acrylic acid-based monomer, it is difficult to exhibit the sufficient antibacterial effect. When the polymerizable antibacterial monomer is included in an amount of more than 1 part by weight with respect to 100 parts by weight of the acrylic acid-based monomer, the radical polymerization reaction may be delayed due to the hydroxy group (OH) included in the polymerizable antibacterial monomer, and accordingly, it is apprehended that sufficient polymerization may not occur or the polymerization time may be prolonged due to reduced polymerization during polymerization of the acrylic acid-based monomer.

More specifically, the polymerizable antibacterial monomer is included in the crosslinked polymer in an amount of 0.2 parts by weight or more, 0.3 parts by weight or more, 0.4 parts by weight or more, or 0.5 parts by weight or more, and 1 part by weight or less with respect to 100 parts by weight of the acrylic acid-based monomer. At this time, in terms of improving antibacterial property and absorption performances of the superabsorbent polymer, the polymerizable antibacterial monomer is preferably included in the crosslinked polymer in an amount of 0.2 parts by weight to 1 part by weight with respect to 100 parts by weight of the acrylic acid-based monomer.

In this regard, the polymerizable antibacterial monomer is included in the crosslinked polymer in an amount of 0.1 part by weight to 1 part by weight with respect to 100 parts by weight of the acrylic acid-based monomer, which means that the polymerizable antibacterial monomer is used in an amount of 0.1 part by weight to 1 part by weight with respect to 100 parts by weight of the acrylic acid-based monomer during the preparation of the crosslinked polymer. This may be confirmed by whether or not the antibacterial monomer is detected when examining the residual monomer of the superabsorbent polymer. In the superabsorbent polymer, the antibacterial monomer was not detected after preparation, which may indicate that all of the used antibacterial monomer was consumed in the polymerization with the acrylic acid-based monomer.

Meanwhile, the acrylic acid-based monomer is a compound represented by the following Formula 2:

[Formula 2] R-COOM'

in Formula 2,

R is an alkenyl group containing an unsaturated bond and having 2 to 5 carbon atoms, and
M' is a hydrogen atom, a monovalent or divalent metal, an ammonium group, or an organic amine salt.

Preferably, the monomer may include one or more selected from the group consisting of (meth)acrylic acid, and a monovalent (alkali) metal salt thereof, a divalent metal salt thereof, an ammonium salt thereof, and an organic amine salt thereof.

As described, when (meth)acrylic acid and/or a salt thereof may be used as the acrylic acid-based monomer, it is advantageous in that a superabsorbent polymer having improved absorbency may be obtained.

Further, as used herein, the term 'crosslinking agent' is used to distinguish it from an additional crosslinking agent which mainly crosslinks the surface of the superabsorbent polymer particle, described later, and functions to polymerize the acrylic acid-based monomers and polymerizable antibacterial monomers by crosslinking unsaturated bonds thereof. The crosslinking in the above step is performed regardless of surface or internal crosslinking. However, when the additional crosslinking process of the superabsorbent polymer particles to be described later is performed, the particle surface of the superabsorbent polymer finally prepared has a crosslinked structure by the additional crosslinking agent, and the inside thereof has a crosslinked structure by the above crosslinking agent. Therefore, since the additional crosslinking agent mainly serves to crosslink the surface of the superabsorbent polymer, it may serve as a surface crosslinking agent, and the above crosslinking agent, distinguished from the second crosslinking agent, may serve as an internal crosslinking agent.

As the crosslinking agent, any compound is possible as long as it enables introduction of crosslinkage during polymerization of the acrylic acid-based monomer. Non-limiting examples of the crosslinking agent may include multifunctional crosslinking agents, such as N,N'-methylenebisacrylamide, trimethylolpropane tri(meth)acrylate, ethylene glycol di(meth)acrylate, polyethylene glycol (meth)acrylate, polyethylene glycol di(meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaerythritol pentaacrylate, glycerin tri(meth)acrylate, pentaerythritol tetraacrylate, triarylamine, ethylene glycol diglycidyl ether, propylene glycol, glycerin, or ethylene carbonate, which may be used alone or in combination of two or more thereof, but are not limited thereto.

Preferably, as the crosslinking agent, polyalkylene glycol di(meth)acrylate-based compounds, such as polyethylene glycol (meth)acrylate, polyethylene glycol di(meth)acrylate, propylene glycol di(meth)acrylate, or polypropylene glycol (meth)acrylate may be used.

The crosslinking polymerization of the acrylic acid-based monomer in the presence of the crosslinking agent may be performed by thermal polymerization, photopolymerization, or hybrid polymerization in the presence of a polymerization initiator, as needed, a thickener, a plasticizer, a preservation stabilizer, an antioxidant, etc., and a detailed description thereof will be described later.

Further, the superabsorbent polymer may be in the form of particles having a particle size of 850 μm or less, for example, about 150 μm to about 850 μm. In this regard, the particle size may be measured in accordance with the European Disposables and Nonwovens Association (EDANA) standard WSP 220.3 method. Here, when the superabsorbent polymer includes a large amount of fine particles having a particle size of less than 150 μm, general physical properties of the superabsorbent polymer may be deteriorated, which is not preferred.

Meanwhile, the superabsorbent polymer includes a crosslinked layer formed on the crosslinked polymer by additionally crosslinking the crosslinked polymer via the additional crosslinking agent. Here, the crosslinked layer is mainly formed on at least part of the surface of the superabsorbent polymer particles, and the crosslinked polymer in the superabsorbent polymer is crosslinked by the additional crosslinking agent. This is to increase the surface crosslinking density of the superabsorbent polymer particles. As described, when the superabsorbent polymer further includes the structure in which at least part of the superabsorbent polymer particles is crosslinked by the additional crosslinking agent, it may have a structure in which the external crosslinking density is higher than the internal crosslinking density.

As the additional crosslinking agent, additional crosslinking agents which have been used in the preparation of the superabsorbent polymer may be used without particular limitation. For example, the additional crosslinking agent may include one or more polyols selected from the group consisting of ethylene glycol, propylene glycol, 1,3-propanediol, 1,4-butanediol, 1,6-hexanediol, 1,2-hexanediol, 1,3-hexanediol, 2-methyl-1,3-propanediol, 2,5-hexanediol, 2-methyl-1,3-pentanediol, 2-methyl-2,4-pentanediol, tripropylene glycol, and glycerol; one or more carbonate-based compounds selected from the group consisting of ethylene carbonate, propylene carbonate, and glycerol carbonate; epoxy compounds such as ethylene glycol diglycidyl ether, etc.; oxazoline compounds such as oxazolidinone, etc.; polyamine compounds; oxazoline compounds; mono-, di-, or polyoxazolidinone compounds; or cyclic urea compounds, etc.

Specifically, as the additional crosslinking agent, one or more, two or more, or three or more of the above-described additional crosslinking agents may be used. For example, ethylene carbonate-propylene carbonate (ECPC), propylene glycol and/or glycerol carbonate may be used.

Meanwhile, the superabsorbent polymer may exhibit the antibacterial property against at least one of Gram-negative bacteria and Gram-positive bacteria, as described above. More specifically, the superabsorbent polymer may exhibit the antibacterial property against one or more bacteria classified as Gram-positive bacteria. Alternatively, the superabsorbent polymer may exhibit the antibacterial property against one or more bacteria classified as Gram-negative bacteria. Alternatively, the superabsorbent polymer may exhibit the antibacterial property against one or more bacteria classified as Gram-negative bacteria and one or more bacteria classified as Gram-positive bacteria.

Here, the "exhibiting the antibacterial property against specific bacteria" means that the number of bacteria cultured after allowing a superabsorbent polymer to be tested for the antibacterial property to absorb artificial urine inoculated with test bacteria is remarkably reduced, as compared to the number of reference bacteria cultured after allowing a superabsorbent polymer without the antibacterial material to absorb artificial urine inoculated with test bacteria. Specifically, it means that the bacteriostatic reduction rate (%) calculated by the following Equation 1 according to the antibacterial property test to be described later is 50% or more.

[Equation 1]

$$\text{Bacteriostatic reduction rate (\%)} = (1 - C_{sample} / C_{Reference}) * 100$$

in Equation,

$C_{sample}$ represents a concentration (Co) of microorganisms in a culture medium of a superabsorbent polymer with the antibacterial material, and
$C_{Reference}$ represents a concentration (Co) of microorganisms in a culture medium of a superabsorbent polymer of Comparative Example 1 without the antibacterial material.

More preferably, the "exhibiting the antibacterial property against specific bacteria" means that the bacteriostatic reduction rate (%) calculated by Equation 1 is 90% to 100%. In one embodiment, the bacteriostatic reduction rate (%) of the superabsorbent polymer, calculated by Equation 1, may be 90% or more, 95% or more, 99% or more, 99% or more, 99.09% or more, 99.18% or more, 99.86% or more, 99.91 % or more, 99.93% or more, or 99.96% or more, and 100% or less.

Meanwhile, the Gram-positive bacteria collectively refer to bacteria that are stained purple when stained by the Gram staining method. The cell wall of Gram-positive bacteria consists of several layers of peptidoglycan. After staining with a basic dye such as crystal violet, even when the Gram-positive bacteria are treated with ethanol, they are not decolorized and remain colored purple. Bacteria categorized as the Gram-positive bacteria include *Enterococcus faecalis, Staphylococcus aureus, Streptococcus pneumoniae, Enterococcus faecium, Lactobacillus lactis,* etc.

Further, the Gram-negative bacteria collectively refer to bacteria that are stained red when stained by the Gram staining method, and have outer membrane consisting of lipopolysaccharides, lipoproteins, and other complex polymer materials instead of the cell wall having a relatively small amount of peptidoglycan, as compared to the Gram-positive bacteria. Thus, after staining with a basic dye such as crystal violet, even when the Gram-negative bacteria are treated with ethanol, they are decolorized, and when they are counter-stained with a red dye such as safranin, they appear red. Bacteria categorized as the Gram-negative bacteria include *Proteus mirabilis, Escherichia coli, Salmonella typhi, Pseudomonas aeruginosa, Vibrio cholerae,* etc.

Therefore, since the Gram-positive bacteria and Gram-negative bacteria may cause various diseases upon contacting, and may also cause secondary infection in critically ill patients with weak immunity, it is preferable that a single antibacterial agent is used to exhibit antibacterial properties against both Gram-positive bacteria and Gram-negative bacteria.

Preferably, the superabsorbent polymer may exhibit antibacterial properties against both the Gram-negative bacteria and the Gram-positive bacteria. In this regard, the Gram-negative bacteria against which the superabsorbent polymer exhibits the antibacterial property may be *Proteus Mirabilis* or *Escherichia coli,* and the Gram-positive bacteria against which the superabsorbent polymer exhibits the antibacterial property may be *Enterococcus faecalis,* but is not limited thereto.

Specifically, the superabsorbent polymer may exhibit a 30-min centrifuge retention capacity (CRC) of 20 g/g to 45 g/g for physiological saline (0.9 wt% aqueous sodium chloride solution), as measured according to the EDANA method WSP 241.3. When the centrifuge retention capacity (CRC) is less than 20 g/g, the ability to retain a liquid after absorption is reduced, and thus the superabsorbent polymer is not suitable for application to hygiene products. More specifically, the centrifuge retention capacity (CRC) of the superabsorbent polymer may be 20 g/g or more, 25 g/g or more, 30 g/g or more, 33 g/g or more, 33.5 g/g or more, 34 g/g or more, 34.2 g/g or more, 34.5 g/g or more, 37 g/g or more, 37.1 g/g or more, or 37.6 g/g or more, and 45 g/g or less, 43 g/g or less, 41 g/g or less, 39 g/g or less, 38 g/g or less, or 37.8 g/g or less.

Accordingly, the above-described superabsorbent polymer including a predetermined amount of the polymerizable antibacterial monomer in the crosslinked polymer may have a centrifuge retention capacity (CRC) of 20 g/g to 45 g/g, and at the same time, may exhibit the excellent antibacterial property.

**Method of preparing superabsorbent polymer**

Meanwhile, the superabsorbent polymer may be prepared by the following preparation method:

preparing a monomer composition by mixing an acrylic acid-based monomer including acidic groups, a polymerizable antibacterial monomer represented by Formula 1, a crosslinking agent, and a polymerization initiator (step 1);
forming a water-containing gel polymer by polymerizing the monomer composition (step 2); and
drying, pulverizing, and classifying the water-containing gel polymer (step 3).

First, to prepare the superabsorbent polymer of one embodiment, the step 1 of preparing the monomer composition by mixing the acrylic acid-based monomer including acidic groups, the polymerizable antibacterial monomer represented by Formula 1, a basic material, the crosslinking agent, and the polymerization initiator is performed.

Here, more detailed descriptions of the acrylic acid-based monomer, the polymerizable antibacterial monomer, and the crosslinking agent are the same as described above, and in the step 1, at least part of the acidic groups of the acrylic acid-based monomer is neutralized by mixing with the basic material. Therefore, the monomer composition may

include the acrylic acid-based monomer of which acidic groups are at least partially neutralized.

Here, as the basic material capable of neutralizing the acrylic acid-based monomer, an alkali material such as sodium hydroxide, potassium hydroxide, ammonium hydroxide, etc. may be used. Further, the basic material may be added to the monomer composition as an aqueous solution of being dissolved in water.

Further, a degree of neutralization of the acrylic acid-based monomer, i.e., a content (mol%) of the neutralized acrylic acid-based monomer based on the total mole of acrylic acid-based monomer used in the preparation of the crosslinked polymer may be 40 mol% to 95 mol%, or 40 mol% to 80 mol%, or 45 mol% to 75 mol%. An excessively high degree of neutralization renders the neutralized monomers precipitated, and thus polymerization may not occur readily, whereas an excessively low degree of neutralization not only greatly deteriorates absorbency of the polymer but also endows the polymer with hard-to-handle properties, such as of elastic rubber.

Further, in the monomer composition, the polymerizable antibacterial monomer may be used in an amount of 0.1 part by weight to 1 part by weight with respect to 100 parts by weight of the acrylic acid-based monomer. When the polymerizable antibacterial monomer is used in an amount of less than 0.1 part by weight with respect to 100 parts by weight of the acrylic acid-based monomer, it is difficult to exhibit the sufficient antibacterial effect, and when the polymerizable antibacterial monomer is used in an amount of more than 1 part by weight with respect to 100 parts by weight of the acrylic acid-based monomer, the radical polymerization reaction may be delayed due to the hydroxy group (OH) included in the polymerizable antibacterial monomer, and accordingly, it is apprehended that sufficient polymerization may not occur or the polymerization time may be prolonged due to reduced polymerization of the acrylic acid-based monomer.

More specifically, the polymerizable antibacterial monomer may be used in an amount of 0.2 parts by weight or more, 0.3 parts by weight or more, 0.4 parts by weight or more, or 0.5 parts by weight or more, and 1 part by weight or less with respect to 100 parts by weight of the acrylic acid-based monomer.

Further, in the monomer composition, the crosslinking agent is included in an amount of 0.01 part by weight to 1 part by weight with respect to 100 parts by weight of the acrylic acid-based monomer, thereby crosslinking the polymerized polymer. When the amount of the crosslinking agent is less than 0.01 part by weight, the improvement effect due to crosslinking is insignificant. When the amount of the crosslinking agent is more than 1 part by weight, absorbency of the superabsorbent polymer may be reduced. More specifically, the crosslinking agent may be included in an amount of 0.2 parts by weight or more, 0.3 parts by weight or more, 0.4 parts by weight or more, or 0.5 parts by weight or more, and 1 parts by weight or less with respect to 100 parts by weight of the acrylic acid-based monomer.

Further, the polymerization initiator may be appropriately selected depending on a polymerization method. When a thermal polymerization method is used, a thermal polymerization initiator is used. When a photopolymerization method is used, a photopolymerization initiator is used. When a hybrid polymerization method (a method of using both heat and light) is used, both the thermal polymerization initiator and the photopolymerization initiator are used. However, even though the photopolymerization method is used, a certain amount of heat is generated by light irradiation such as UV irradiation, etc., and is also generated with the polymerization reaction which is an exothermic reaction. Therefore, the thermal polymerization initiator may be further used.

As the photopolymerization initiator, any compound capable of forming radicals by a light such as UV may be used without limitations in view of its composition.

For example, one or more selected from the group consisting of benzoin ether, dialkyl acetophenone, hydroxyl alkylketone, phenyl glyoxylate, benzyl dimethyl ketal, acyl phosphine, and $\alpha$-aminoketone may be used as the photopolymerization initiator. Meanwhile, specific examples of acyl phosphine may include diphenyl(2,4,6-trimethylbenzoyl)phosphine oxide, phenylbis(2,4,6-trimethylbenzoyl)phosphine oxide, ethyl (2,4,6-trimethylbenzoyl) phenylphosphinate, etc. More various photopolymerization initiators are well disclosed in "UV Coatings: Basics, Recent Developments and New Application (Elsevier, 2007)" written by Reinhold Schwalm, p115, however, they are not limited to the above described examples.

The photopolymerization initiator may be included in an amount of 0.001 part by weight to 1 part by weight with respect to 100 parts by weight of the acrylic acid-based monomer. When the amount of the photopolymerization initiator is less than 0.001 part by weight, the polymerization rate may become slow, and when the amount of the photopolymerization initiator is more than 1 part by weight, a molecular weight of the superabsorbent polymer becomes small and its physical properties may become uneven. More specifically, the photopolymerization initiator may be included in an amount of 0.005 parts by weight or more, or 0.00625 parts by weight or more, and 0.5 parts by weight or less, or 0.3 parts by weight or less with respect to 100 parts by weight of the acrylic acid-based monomer.

Further, when the thermal polymerization initiator is further included as the polymerization initiator, one or more selected from the group consisting of a persulfate-based initiator, an azo-based initiator, hydrogen peroxide, and ascorbic acid may be used as the thermal polymerization initiator. Specific examples of the persulfate-based initiator may include sodium persulfate ($Na_2S_2O_8$), potassium persulfate ($K_2S_2O_8$), ammonium persulfate ($(NH_4)_2S_2O_8$), etc., and examples of the azo-based initiator may include 2,2-azobis-(2-amidinopropane)dihydrochloride, 2,2-azobis-(N,N-dimethylene)isobutyramidine dihydrochloride, 2-(carbamoylazo)isobutyronitrile, 2,2-azobis[2-(2-imidazolin-2-yl)propane]dihydrochloride, 4,4-azobis-(4-cyanovaleric acid), etc. More various thermal polymerization initiators are well disclosed in 'Principle of Polymerization(Wiley, 1981)', written by Odian, p 203, however, the thermal polymerization initiator is not limited to the above-described examples.

The thermal polymerization initiator may be included in an amount of 0.001 part by weight to 1 part by weight with respect to 100 parts by weight of the acrylic acid-based monomer. When the amount of the thermal polymerization initiator is less than 0.001 part by weight, additional thermal polymerization hardly occurs, and thus the addition effect of the thermal polymerization initiator may be insignificant. When the amount of the thermal polymerization initiator is more than 1 part by weight, the molecular weight of the superabsorbent polymer may become low and its physical properties may become uneven. More specifically, the thermal polymerization initiator may be included in an amount of 0.005 parts by weight or more, or 0.01 parts by weight or more, 0.1 parts by weight or more, or 0.125 parts by weight or more, and 0.5 parts by weight or less, or 0.3 parts by weight or less with respect to 100 parts by weight of the acrylic acid-based monomer.

Further, the monomer composition may further include one or more kinds of additives such as a surfactant, a thickener, a plasticizer, a preservation stabilizer, an antioxidant, etc., as needed.

The above-described monomer composition may be prepared in the form of a solution of being dissolved in a solvent.

As the solvent to be applicable, any solvent may be used without limitations in view of composition as long as it is able to dissolve the above components, and for example, one or more selected from water, ethanol, ethylene glycol, diethylene glycol, triethylene glycol, 1,4-butanediol, propylene glycol, ethylene glycol monobutyl ether, propylene glycol monomethyl ether, propylene glycol monomethyl ether acetate, methyl ethyl ketone, acetone, methyl amyl ketone, cyclohexanone, cyclopentanone, diethylene glycol monomethyl ether, diethylene glycol ethylether, toluene, xylene, butyrolactone, carbitol, methyl cellosolve acetate, and N,N-dimethylacetamide may be used in combination. The solvent may be included in the remaining amount excluding the above-described components, with respect to the total amount of the monomer composition.

Next, the step 2 of forming a water-containing gel polymer by performing thermal polymerization or photopolymerization of the monomer composition is performed.

Here, the method of performing thermal polymerization or photopolymerization is not particularly limited in view of constitution, as long as it is a common method capable of forming the water-containing gel polymer by polymerizing the monomer composition.

For example, the photopolymerization may be performed by irradiating UV with an intensity of 3 mW to 30 mW, or 10 mW to 20 mW at a temperature of 60°C to 90°C, or 70°C to 80°C. When the photopolymerization may be performed under these conditions, it is possible to form the crosslinked polymer with higher polymerization efficiency.

Further, when the photopolymerization is performed, it may be performed in a reactor equipped with a movable conveyor belt or in a stainless steel container with a predetermined size, but the above-described polymerization method is an example only, and the present invention is not limited to the above-described polymerization methods.

Further, as described above, when the photopolymerization is performed in a reactor equipped with a movable conveyor belt, the obtained water-containing gel polymer may be usually a sheet-like water-containing gel polymer having a width of the belt. In this case, a thickness of the polymer sheet may vary depending on the concentration of the monomer composition fed thereto and the feeding speed, and usually, it is preferable to supply the monomer composition such that a sheet-like polymer having a thickness of about 0.5 cm to about 5 cm may be obtained. When the monomer composition is supplied to such an extent that the thickness of the sheet-like polymer becomes too thin, it is undesirable because the production efficiency is low, and when the thickness of the sheet-like polymer is more than 5 cm, the polymerization reaction may not evenly occur over the entire thickness because of the excessive thickness.

Further, a water content of the water-containing gel polymer obtained from the step 2 may be about 40% by weight to about 80% by weight with respect to the total weight of the water-containing gel polymer. Meanwhile, the "water content" throughout the present specification means a weight occupied by water with respect to the total weight of the water-containing gel polymer, which may be a value obtained by subtracting the weight of the dried polymer from the weight of the water-containing gel polymer. Specifically, the water content may be defined as a value calculated by measuring the weight loss due to evaporation of moisture in the polymer during a process of drying by raising the temperature of the polymer through infrared heating. At this time, the water content is measured under the following drying conditions: the temperature is increased from room temperature to about 180°C and then the temperature is maintained at 180°C, and the total drying time is set to 20 minutes, including 5 minutes for the temperature rising step.

Meanwhile, after the preparation of the water-containing gel polymer, a process of coarsely pulverizing the prepared water-containing gel polymer may be optionally performed, before subsequent drying and pulverizing processes.

The coarse pulverization process is a process for increasing the drying efficiency in the subsequent drying process and controlling the particle size of the prepared superabsorbent polymer powder. In this regard, a pulverizer used here is not limited to its configuration, and specifically, it may include any one selected from the group consisting

of a vertical pulverizer, a turbo cutter, a turbo grinder, a rotary cutter mill, a cutter mill, a disc mill, a shred crusher, a crusher, a meat chopper, and a disc cutter, but is not limited to the above-described examples.

The coarse pulverization process may be performed, for example, such that the water-containing gel polymer may have a particle size of about 1 mm to about 10 mm. Pulverization of the water-containing gel polymer to a particle size of smaller than 1 mm is not technically easy due to the high water content of the water-containing gel polymer, and an agglomeration phenomenon between the pulverized particles may occur. Meanwhile, when the water-containing gel polymer is pulverized to a particle size of larger than 10 mm, the effect of increasing the efficiency in the subsequent drying step may be insignificant.

Next, the step 3 of forming the superabsorbent polymer including the crosslinked polymer by drying, pulverizing, and classifying the water-containing gel polymer prepared in the step 2 is performed.

The drying method may be selected and used without limitation in view of constitution, as long as it is commonly used in the process of drying the water-containing gel polymer. Specifically, the drying step may be performed by a method such as hot air supply, infrared irradiation, microwave irradiation, ultraviolet irradiation, etc.

Specifically, the drying may be performed at a temperature of about 100°C to about 250°C. When the drying temperature is lower than 100°C, it is apprehended that the drying time becomes too long and the physical properties of the superabsorbent polymer finally formed may be deteriorated. When the drying temperature is higher than 250°C, only the polymer surface is excessively dried, and thus fine particles may be generated during the subsequent pulverization process and the physical properties of the superabsorbent polymer finally formed may be deteriorated. Therefore, the drying may be preferably performed at a temperature of 100°C or higher, or 110°C higher, and 200°C or lower, or 180°C or lower.

The polymer after such a drying step may have a water content of about 5% by weight to about 10% by weight.

Meanwhile, after the drying process, a pulverization process is performed. The pulverization process may be performed such that the polymer powder, i.e., the superabsorbent polymer particle has a particle size of about 150 μm to about 850 μm. A pulverizer which is used for pulverization to such a particle size may include specifically a pin mill, a hammer mill, a screw mill, a roll mill, a disc mill, a jog mill, etc., but the present invention is not limited thereto.

After the above pulverization step, to manage the physical properties of the superabsorbent polymer to be finally commercialized, the pulverized polymer powder may be further subjected to a classifying process according to the particle size.

The superabsorbent polymer resulting from the above process may have a fine powder form including the crosslinked polymer obtained by crosslinking polymerization of the acrylic acid-based monomer and the polymerizable antibacterial monomer via the crosslinking agent. Specifically, the superabsorbent polymer may have a fine powder form having a particle size of 150 μm to 850 μm.

Next, the step of performing surface crosslinking of the superabsorbent polymer prepared in the step 3 by heat treatment in the presence of an additional crosslinking agent may be further included. Through the above step, the superabsorbent polymer further including a surface crosslinked layer on the crosslinked polymer, in which the crosslinked polymer is further crosslinked via the additional crosslinking agent, may be prepared. In this regard, the description of the additional crosslinking agent refers to the above description.

The surface crosslinking is a step of increasing the crosslinking density in the vicinity of the surface of the superabsorbent polymer in relation to the internal crosslinking density of particles. In general, the additional crosslinking agent is applied to the surface of the polymer. Therefore, this reaction occurs on the surface of the polymer particle, which improves the crosslinking property on the surface of the particle without substantially affecting the interior of the particle. Thus, the surface-crosslinked superabsorbent polymer has a higher level of crosslinking in the vicinity of the surface than in the interior.

Further, the additional crosslinking agent may be used in an amount of about 0.001 part by weight to about 5 parts by weight with respect to 100 parts by weight of the superabsorbent polymer. For example, the additional crosslinking agent may be used in an amount of 0.005 parts by weight or more, 0.01 part by weight or more, or 0.05 parts by weight or more, and 5 parts by weight or less, 4 parts by weight or less, or 3 parts by weight or less with respect to 100 parts by weight of the superabsorbent polymer. It is possible to prepare a superabsorbent polymer exhibiting excellent general absorption properties by controlling the amount of the additional crosslinking agent in the above-described range.

Further, a method of mixing the additional crosslinking agent with the superabsorbent polymer is not limited in view of its constitution. For example, a method of feeding the additional crosslinking agent and the superabsorbent polymer to a reactor and mixing them with each other, a method of spraying the additional crosslinking agent onto the superabsorbent polymer, or a method of mixing the superabsorbent polymer and the additional crosslinking agent while continuously feeding them to a mixer which is continuously operated may be used.

The surface crosslinking may be carried out by heating the superabsorbent polymer powder, to which the additional crosslinking agent has been added, at a temperature of about 80°C to about 220°C for about 15 minutes to about 100 minutes. When the crosslinking reaction temperature is lower than 80°C, a sufficient surface crosslinking reaction may not occur, and when the crosslinking reaction temperature is higher than 220°C, an excessive surface

crosslinking reaction may occur. Further, when the crosslinking reaction time is as short as less than 15 minutes, a sufficient crosslinking reaction may not occur, and when the crosslinking reaction time exceeds 100 minutes, the crosslinking density of the particle surface is excessively increased due to excessive surface crosslinking reaction, leading to deterioration in the physical properties. More specifically, the surface crosslinking reaction may be carried out by heating at a temperature of 120°C or higher, or 140°C or higher, and 200°C or lower, or 180°C or lower for 20 minutes or more, or 40 minutes or more, and 70 minutes or less, or 60 minutes or less.

A means for raising the temperature for the surface crosslinking reaction is not particularly limited. Heating may be performed by providing a heating medium or by directly providing a heat source. In this regard, the type of the heating medium applicable may be a hot fluid such as steam, hot air, hot oil, or the like. However, the present invention is not limited thereto. The temperature of the heating medium provided may be properly controlled, considering the means of the heating medium, the heating rate, and the target temperature. Meanwhile, as the heat source provided directly, an electric heater or a gas heater may be used, but the present invention is not limited to these examples.

Furthermore, provided is an article including the above-described superabsorbent polymer.

The above article may be one or more selected from the group consisting of absorbent articles, hygiene products, water retaining soil products, water stop materials for the civil engineering and construction, sheets for raising seedling, fresh-keeping agents, materials for poultice, electrical insulators, oral care products, dental products, cosmetic products, and skin products.

In this regard, the hygiene products including the superabsorbent polymer may include paper diapers for children, diapers for adults, or sanitary napkins, etc. Such hygiene products may have the configuration of common hygiene products, except that the above-described superabsorbent polymer of one embodiment is included in an absorbent.

Hereinafter, preferred exemplary embodiments will be provided for better understanding of the present invention. However, the following exemplary embodiments are provided only for illustrating the present invention, but the present invention is not limited thereto.

**Preparation Example A: Preparation of polymerizable antibacterial monomer 1-1**

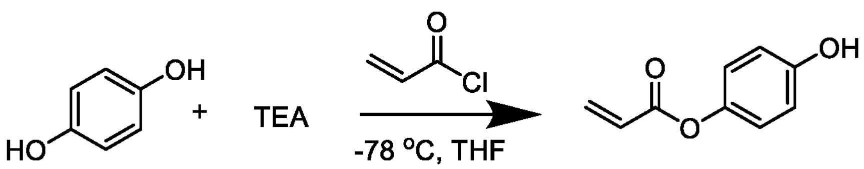


1-1

Hydroquinone (21 g, 19.1 mmol) and triethylamine (TEA, 38.7 g, 38.2 mmol) were placed in a 250 mL round-bottom flask and dissolved in 150 mL of tetrahydrofuran (THF). Then, the temperature of the reactor was lowered to -78°C in an ice bath using dry ice and acetone, and acryloyl chloride (17.3 g, 19.1 mmol) was slowly added dropwise to the reaction solution for 20 minutes. After completion of the reaction, the reaction product was sufficiently diluted in ethyl acetate, and then washed with EtOAc/brine to separate an organic layer. Water was removed with magnesium sulfate, and the passed solution was concentrated under reduced pressure, and purified by a hexane/ethyl acetate column chromatography separation method to obtain a polymerizable antibacterial monomer 1-1 (25.7 g, yield of 82%). $^1H$ NMR spectrum of the obtained polymerizable antibacterial monomer 1-1 is shown in FIG. 1.

$MS[M+H]^+ = 164.05$

$^1H$ NMR (500 MHz, DMSO, δ [ppm]): 6.2 (dd, 1H, $CH_2$=CH), 6.3 (dd, 1H, $CH_2$=CH), 6.5 (dd, 1H, $CH_2$=CH), 6.7 (d, 1H, Ar-H), 6.9 (d, 1H, Ar-H), 9.46 (s, 1H, OH).

**Preparation Example B: Preparation of polymerizable antibacterial monomer 1-2**

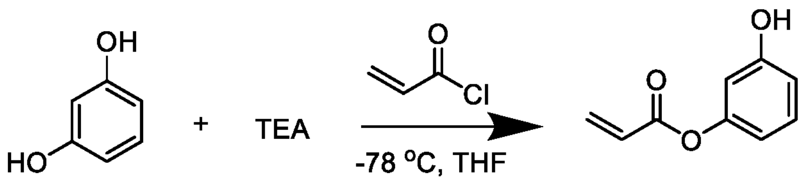


**1-2**

Resorcinol (21 g, 19.1 mmol) and triethylamine (TEA, 38.7 g, 38.2 mmol) were placed in a 250 mL round-bottom flask and dissolved in 150 mL of tetrahydrofuran (THF). Then, the temperature of the reactor was lowered to -78°C in an ice bath using dry ice and acetone, and acryloyl chloride (17.3 g, 19.1 mmol) was slowly added dropwise to the reaction solution for 20 minutes. After completion of the reaction, the reaction product was sufficiently diluted in ethyl acetate, and then washed with EtOAc/brine to separate an organic layer. Water was removed with magnesium sulfate, and the passed solution was concentrated under reduced pressure, and purified by a hexane/ethyl acetate column chromatography separation method to obtain a polymerizable antibacterial monomer 1-2 (22.3 g, yield of 71 %).

$MS[M+H]^+$= 164.05

$^1H$ NMR (500 MHz, DMSO, δ [ppm]): 6.2 (dd, 1H, CH2=CH), 6.3 (dd, 1H, CH2=CH), 6.5 (dd, 1H, CH2=CH), 6.4 (d, 1H, Ar-H), 6.6 (d, 1H, Ar-H), 9.89 (s, 1H, OH).

**Example** - **Preparation of superabsorbent polymer composition**

**Example 1**

**(Step 1)** In a 3L glass container equipped with a stirrer, a nitrogen feeder, and a thermometer, 100 g of acrylic acid, 0.25 g of polyethylene glycol diacrylate (Mn= 575) as a crosslinking agent, 0.00625 g of phenylbis(2,4,6-trimethylbenzoyl)-phenylphosphine oxide as a photoinitiator, 0.125 g by weight of sodium persulfate (SPS) as a thermal initiator, 100 g of a 40% sodium hydroxide solution, and 0.2 g of the polymerizable antibacterial monomer 1-1 prepared in Preparation Example A were added to prepare a monomer composition while feeding nitrogen continuously. At this time, the degree of neutralization of the acrylic acid and the polymerizable antibacterial monomer 1-1 was 70 mol%.

**(Step 2)** Thereafter, the monomer composition was transferred to a stainless steel container having a width of 250 mm, a length of 250 mm, and a height of 30 mm, and then subjected to polymerization by ultraviolet irradiation (dose: 10 $mV/cm^2$) in a UV chamber at 80°C for 60 seconds and aged for 2 minutes to prepare a sheet-type water-containing gel polymer.

**(Step 3)** The prepared water-containing gel polymer was cut into a size of 3 mm * 3 mm, and introduced into a meat chopper to pulverize the polymer, and thus water-containing gel particle crumb having a size of 1 mm to 10 mm was obtained. Then, the obtained crumb was spread on a stainless wire gauze having a hole size of 600 μm at a thickness of about 30 mm, and dried in a hot air oven at 120°C for 11 hours. The dried polymer thus obtained was pulverized using a pulverizer, and classified with a standard sieve according to ASTM to obtain a base polymer having a particle size of 150 μm to 850 μm, which was determined as a superabsorbent polymer.

**Example 2**

A superabsorbent polymer was prepared in the same manner as in Example 1, except that 0.5 g of the polymerizable antibacterial monomer 1-1 prepared in Preparation Example A was used in Example 1.

**Example 3**

A superabsorbent polymer was prepared in the same manner as in Example 1, except that 1.0 g of the polymerizable antibacterial monomer 1-1 prepared in Preparation Example A was used in Example 1.

**Example 4**

A superabsorbent polymer was prepared in the same manner as in Example 1, except that the polymerizable antibacterial monomer 1-2 prepared in Preparation Example B was used, instead of the polymerizable antibacterial monomer 1-1 prepared in Preparation Example A, in Example 1.

**Example 5**

A superabsorbent polymer was prepared in the same manner as in Example 4, except that 0.5 g of the polymerizable antibacterial monomer 1-2 prepared in Preparation Example B was used in Example 4.

**Example 6**

A superabsorbent polymer was prepared in the same manner as in Example 4, except that 1.0 g of the polymerizable antibacterial monomer 1-2 prepared in Preparation Example B was used in Example 4.

**Comparative Example 1**

A superabsorbent polymer was prepared in the same manner as in Example 1, except that the polymerizable antibacterial monomer 1-1 was not used in Example 1.

**Reference Example 1**

A superabsorbent polymer was prepared in the same manner as in Example 1, except that 0.05 g of the polymerizable antibacterial monomer 1-1 prepared in Preparation Example A was used in Example 1.

**Experimental Example 1**

Physical properties of the superabsorbent polymers prepared in Examples and Comparative Examples were evaluated by the following methods, and the results are shown in Table 4 below.
Unless otherwise indicated, the following property evaluation was performed at constant temperature and constant humidity (23±1°C, relative humidity of 50±10%), and physiological saline or brine indicates a 0.9 wt% aqueous sodium chloride solution.

(1) Evaluation of antibacterial property against *E.coli*

Each 2 g of the superabsorbent polymers prepared in Examples and Comparative Examples was put in a 250 cell culture flask, respectively, and then 50 ml of artificial urine, to which a test bacterium, *Escherichia coli (E. coli,* ATCC 25922) was inoculated at 3000±300 CFU/ml, was injected thereto. Thereafter, to allow the superabsorbent polymer to sufficiently absorb the artificial urine solution, they were mixed for about 1 minute. When the polymer sufficiently absorbed the solution, it exhibited a gel form, which was incubated in an incubator (JEIO TECH) at 35°C for 12 hours. To the sample, of which incubation was completed, 150 ml of 0.9 wt% NaCl solution was added, followed by shaking for about 1 minute. This dilution was spread on an agar medium plate. Thereafter, serial dilution was performed for colony counting, and in this procedure, 0.9 wt% NaCl solution was used. Antibacterial performance was determined by calculating a bacteriostatic reduction rate (%) against E. *coli* (ATCC 25922) according to the following Equation 1 after calculating the initial concentration of the bacteria (Co, CFU/mL) based on the dilution concentrations. The results are shown in Table 4 below.

[Equation 1]

$$\text{Bacteriostatic reduction rate (\%)} = (1 - C_{sample} / C_{Reference}) * 100$$

in Equation,

$C_{sample}$ represents a concentration (Co) of microorganisms in a culture medium of the superabsorbent polymer with the antibacterial material, and
$C_{Reference}$ represents a concentration (Co) of microorganisms in a culture medium of the superabsorbent polymer of Comparative Example 1 without the antibacterial material.

At this time, the artificial urine used for the evaluation of the antibacterial property was prepared as follows.
First, each reagent listed in Table 1 below was put into a 1000 mL flask according to the weight described, and the flask was filled with water up to the 1000 mL scale, and mixed to prepare a stock solution. Each reagent listed in Table 2 below was put into a 100 mL flask according to the weight described, and the flask was filled with water up to the 20 mL scale, and mixed to prepare a cationic solution. Each reagent listed in Table 3 below was put into a 100 mL

flask according to the weight described, and the flask was filled with water up to the 100 mL scale, and mixed to prepare a urea/glucose solution.

Next, the prepared stock solution and cationic solution were sterilized at 120°C for 15 minutes using an autoclave, sufficiently cooled to room temperature, and from the prepared urea/glucose solution, impurities were removed using a 0.22 μm syringe filter (Hydrophilic, manufactured by Sartorius stedim).

Thereafter, 940 mL of the stock solution, 10 mL of the cationic solution, and 50 mL of the urea/glucose solution were sufficiently mixed to prepare artificial urine. The prepared artificial urine may be usually stored in a refrigerator and used for 2 weeks, and artificial urine after 2 weeks was not used in the experiment.

[Table 1]

| Stock solution | |
|---|---|
| Reagent name | Weight (g) |
| Sodium chloride | 8.7660 |
| Potassium phosphate dibasic trihydrate | 4.5644 |
| Sodium dihydrogen phosphate (dihydrate) | 1.5602 |
| Ammonium chloride | 2.6745 |
| Sodium sulfate decahydrate | 6.4440 |
| Lactic acid (85% in $H_2O$) | 0.5299 |
| Yeast extract | 20.0000 |

[Table 2]

| Cationic solution | |
|---|---|
| Reagent name | Weight (g) |
| Magnesium chloride(hexahydrate) | 1.2198 |
| Calcium chloride (dihydrate) | 0.8821 |

[Table 3]

| Urea/glucose solution | |
|---|---|
| Reagent name | Weight (g) |
| Urea | 36.0360 |
| D-glucose | 0.1802 |

(2) Centrifuge retention capacity (CRC)

Centrifuge retention capacity by absorption capacity under no load was measured for the superabsorbent polymers of Examples and Comparative Examples in accordance with the European Disposables and Nonwovens Association (EDANA) standard WSP 241.3.

In detail, after uniformly introducing $W_0$(g) (about 0.2 g) of the superabsorbent polymer in a nonwoven fabric-made bag and sealing the same, it was immersed in physiological saline (0.9 wt% aqueous sodium chloride solution) at room temperature. After 30 minutes, the bag was drained by using a centrifuge at 250 G for 3 minutes, and then the weight $W_2$(g) of the bag was measured. Further, after carrying out the same operation without using the polymer, the weight $W_1$(g) of the bag was measured. CRC (g/g) was calculated using each obtained weight according to the following Equation, and the results are shown in Table 4 below.

[Equation 2]

$$CRC\ (g/g) = \{[W_2(g) - W_1(g)]/W_0(g)\} - 1$$

in Equation 2,
$W_0(g)$ is the initial weight (g) of the superabsorbent polymer,
$W_1(g)$ is the weight of the bag, which was measured after the bag without the superabsorbent polymer was immersed in physiological saline for 30 minutes, and then drained using a centrifuge at 250 G for 3 minutes, and
$W_2(g)$ is the weight of the bag including the superabsorbent polymer, which was measured after the superabsorbent polymer was immersed in physiological saline at room temperature for 30 minutes, and then drained using a centrifuge at 250 G for 3 minutes.

[Table 4]

| | Kind of antibacterial monomer | Content of antibacterial monomer[1)] | Antibacterial property against *E.coli* | | | Physical properties of superabsorbent polymer |
|---|---|---|---|---|---|---|
| | | | CFU/mL SUE | Log CFU/mL SUE | Bacteriostatic reduction rate (%) | CRC (g/g) |
| Example 1 | 1-1 | 0.2 | 1.0E+04 | 4.01 | 99.18 | 34.5 |
| Example 2 | 1-1 | 0.5 | 1.1E+03 | 3.05 | 99.91 | 37.1 |
| Example 3 | 1-1 | 1.0 | 4.6E+02 | 2.66 | 99.96 | 37.8 |
| Example 4 | 1-2 | 0.2 | 1.1E+04 | 4.06 | 99.09 | 34.2 |
| Example 5 | 1-2 | 0.5 | 1.8E+03 | 3.25 | 99.86 | 37.0 |
| Example 6 | 1-2 | 1.0 | 9.4E+02 | 2.97 | 99.93 | 37.6 |
| Comparative Example 1 | 1-1 | 0 | 1.2E+06 | 6.10 | 0 | 32.7 |
| Reference Example 1 | 1-1 | 0.05 | 8.4E+05 | 5.92 | 32.75 | 33.0 |
| 1) parts by weight with respect to 100 parts by weight of acrylic acid monomer | | | | | | |

Referring to Table 4, it was confirmed that the superabsorbent polymers of Examples exhibited improved centrifuge retention capacity (CRC), and at the same time, excellent antibacterial property against *E.coli* which is one of Gram-negative bacteria, as compared to the superabsorbent polymer of Comparative Example 1, in which the polymerizable antibacterial monomer was not used during polymerization of the acrylic acid-based monomer, and the superabsorbent polymer of Reference Example 1, in which the polymerizable antibacterial monomer was used in an amount of 0.05 parts by weight with respect to 100 parts by weight of the acrylic acid monomer.

**Claims**

1. A superabsorbent polymer comprising a crosslinked polymer of an acrylic acid-based monomer including acidic groups of which at least part is neutralized; a polymerizable antibacterial monomer represented by the following Formula 1; and a crosslinking agent:

[Formula 1]

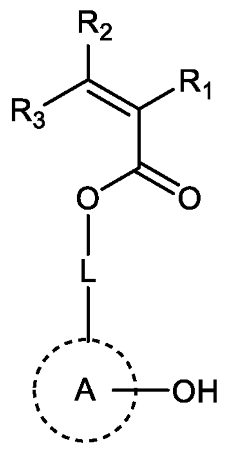

in Formula 1,

$R_1$ to $R_3$ are each independently hydrogen, alkyl having 1 to 4 carbon atoms, or a carboxyl group (COOH),
L is a single bond, or arylene having 6 to 60 carbon atoms, and
A is an aromatic ring having 6 to 60 carbon atoms,

wherein the arylene and the aromatic ring are each independently unsubstituted or substituted with one or more substituents selected from the group consisting of hydroxy, alkyl having 1 to 10 carbon atoms, and alkoxy having 1 to 10 carbon atoms.

**2.** The superabsorbent polymer of claim 1,
wherein $R_1$ to $R_3$ are each independently hydrogen, methyl, or carboxyl group.

**3.** The superabsorbent polymer of claim 1,
wherein L is a single bond, phenylene, or naphthylene.

**4.** The superabsorbent polymer of claim 1,

wherein A is a benzene or naphthalene ring,
wherein the benzene and naphthalene rings are each independently unsubstituted or substituted with 1 to 3 substituents selected from the group consisting of hydroxy, alkyl having 1 to 10 carbon atoms, and alkoxy having 1 to 10 carbon atoms.

**5.** The superabsorbent polymer of claim 1,
wherein the polymerizable antibacterial monomer is represented by any one of the following Formulae 1-1 to 1-3:

[Formula 1-1]

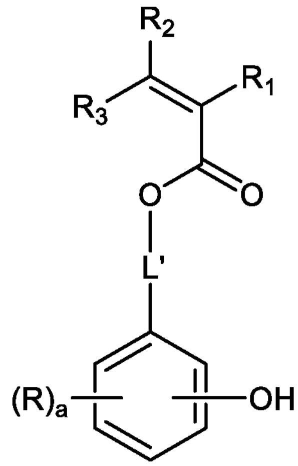

[Formula 1-2]

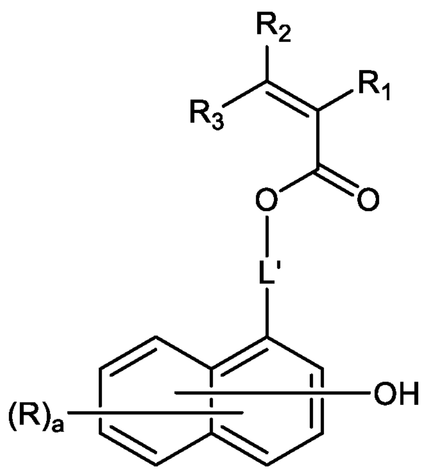

[Formula 1-3]

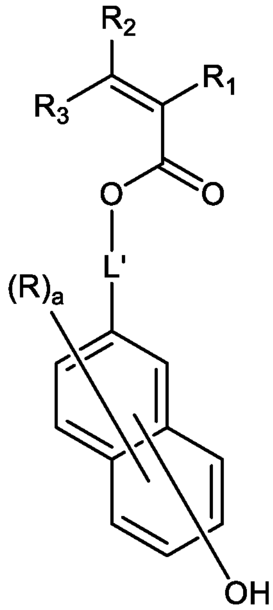

in Formulae 1-1 to 1-3,

L’ is a single bond, phenylene, or naphthylene,
R is hydroxy, alkyl having 1 to 10 carbon atoms, or alkoxy having 1 to 10 carbon atoms,
a is an integer of 0 to 3,
when a is 2 or more, two or more R’s are the same as or different from each other, and
$R_1$ to $R_3$ are as defined in Claim 1.

**6.** The superabsorbent polymer of claim 1,
wherein the polymerizable antibacterial monomer is any one selected from the group consisting of the following compounds:

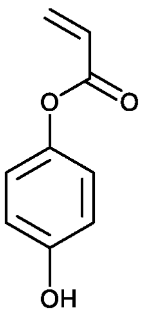
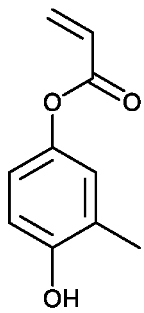
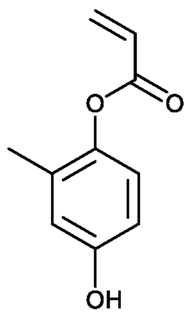
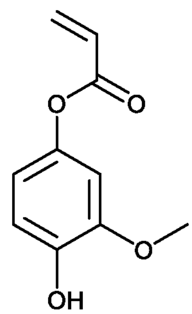

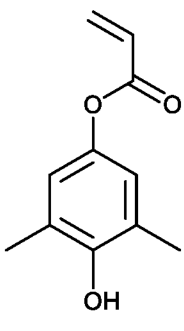
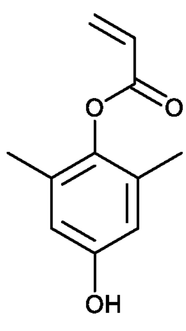
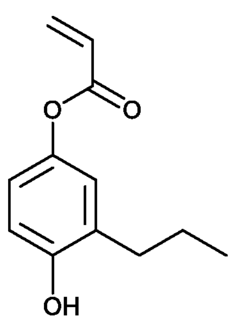

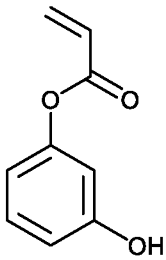
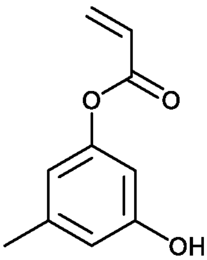
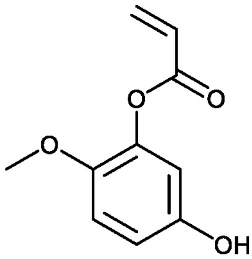
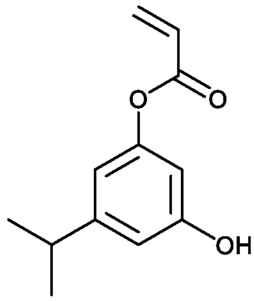

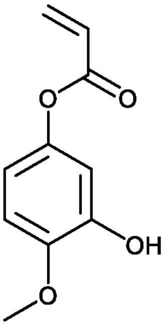
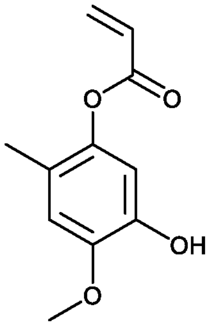
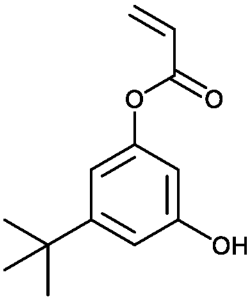
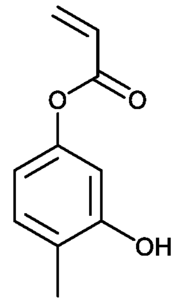

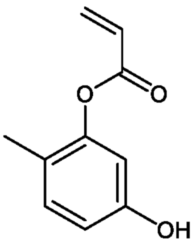
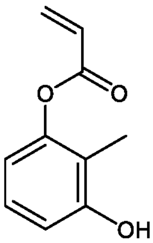
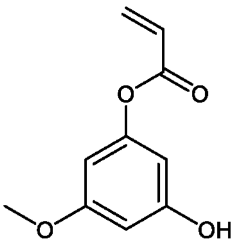
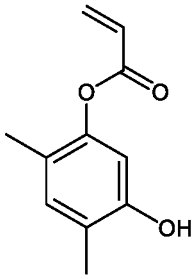

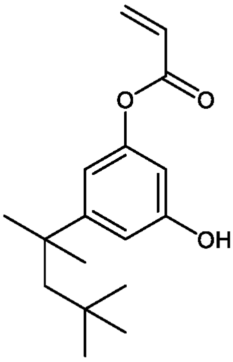
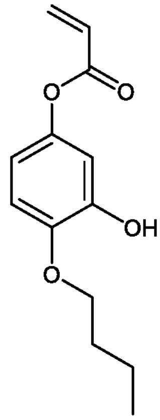

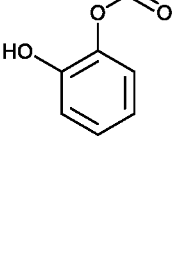
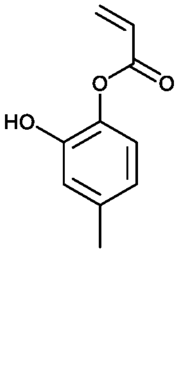
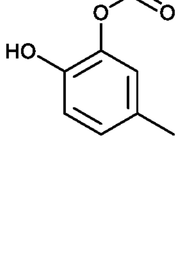
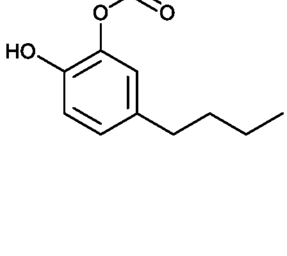

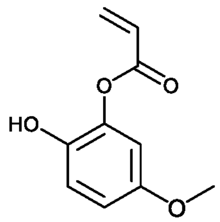
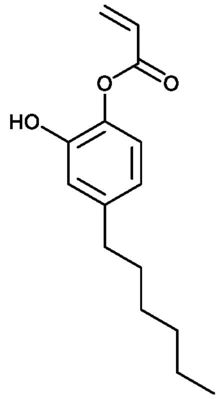
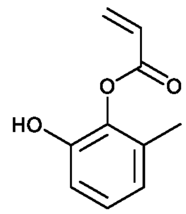
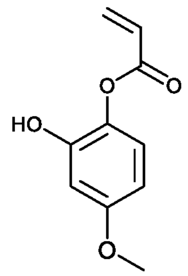

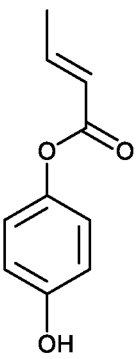
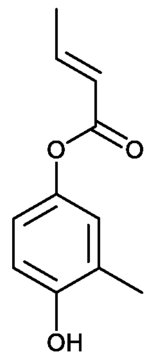
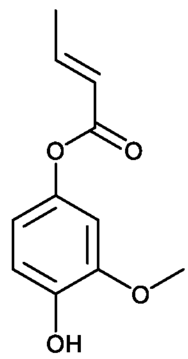
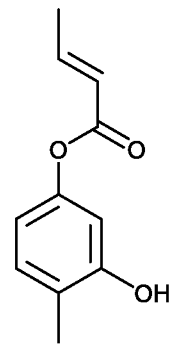

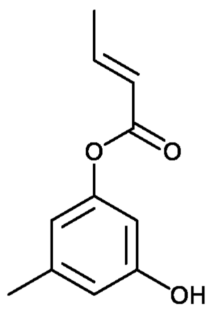
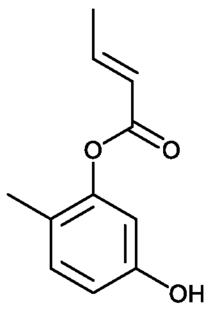
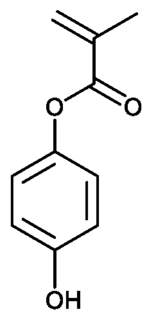
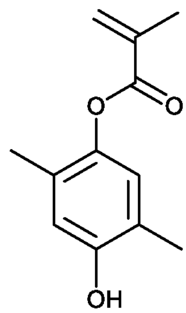

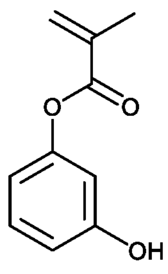
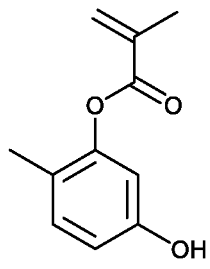
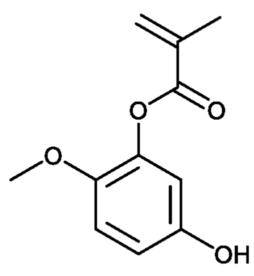
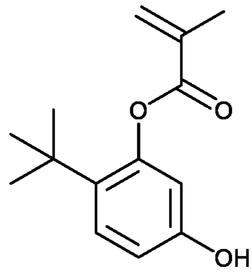

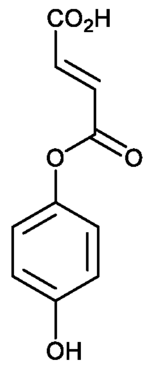
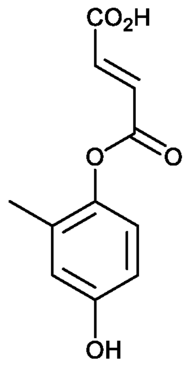
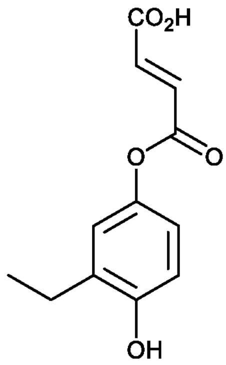
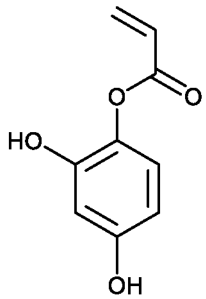
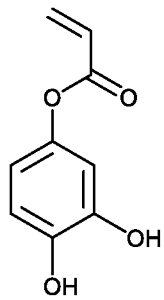
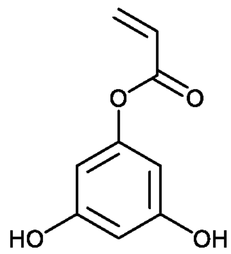
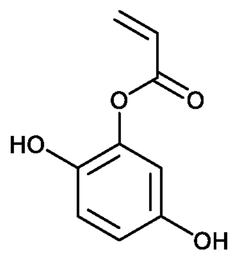
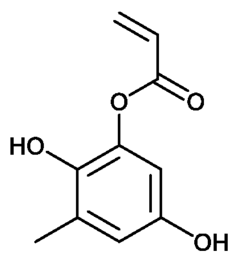
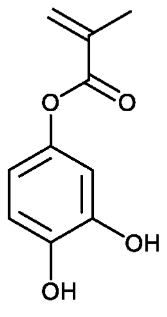
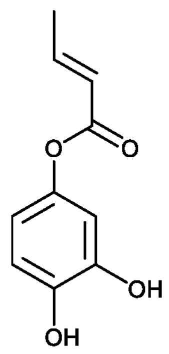
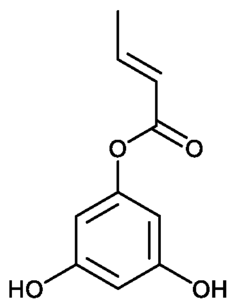
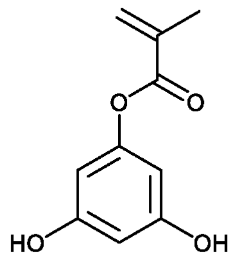
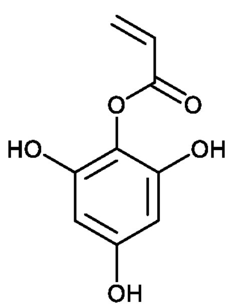
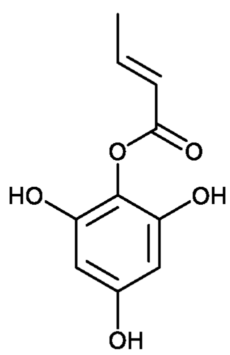
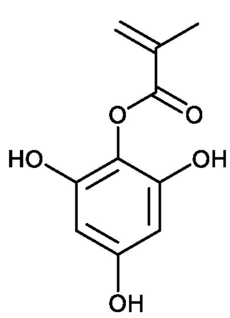
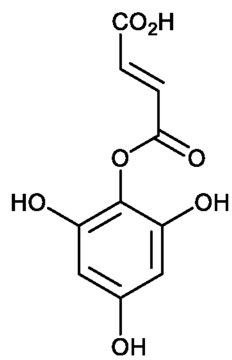

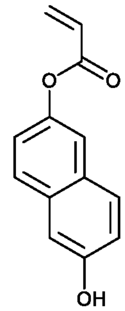
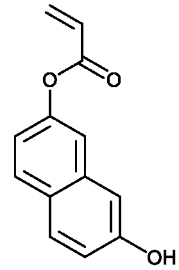
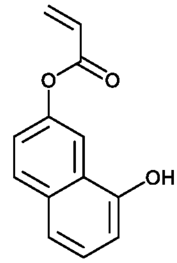
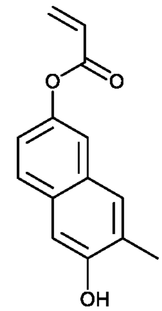
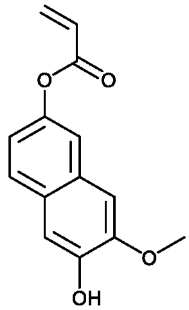
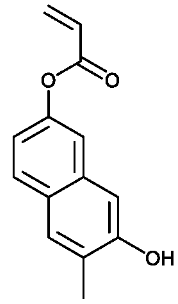
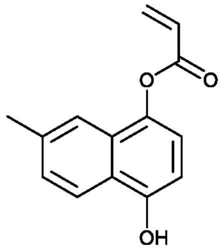
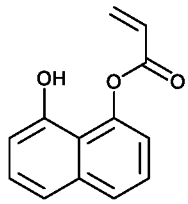
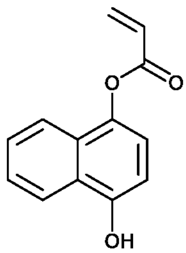
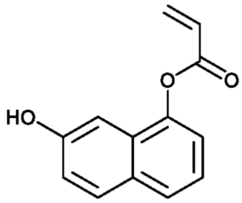
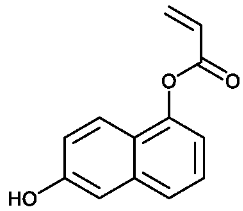
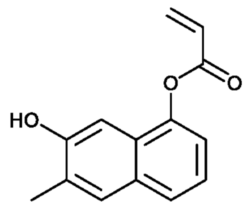
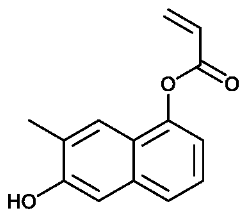
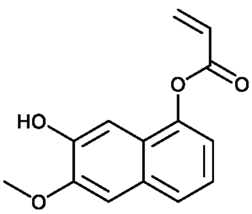
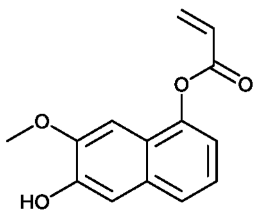
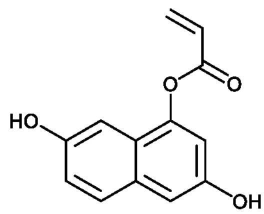
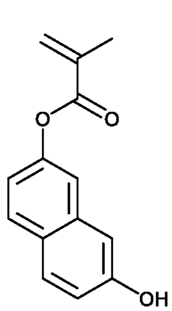
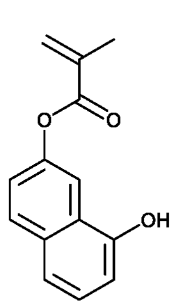
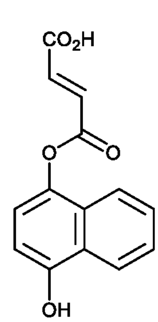
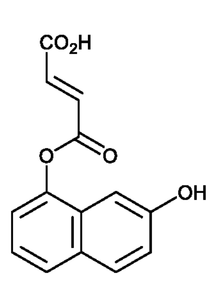

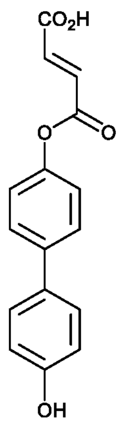 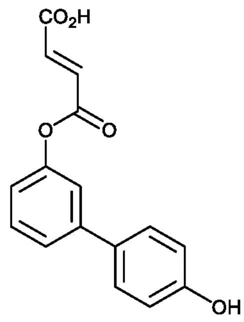

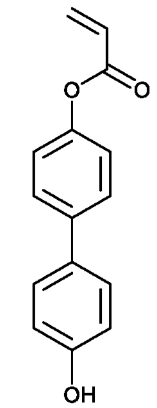 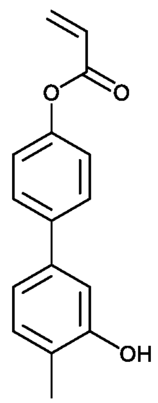 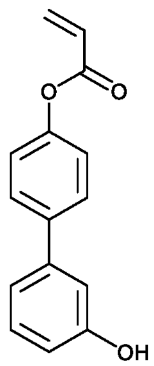 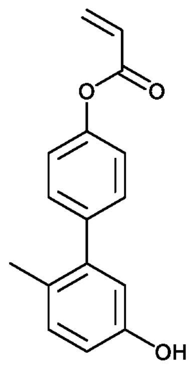

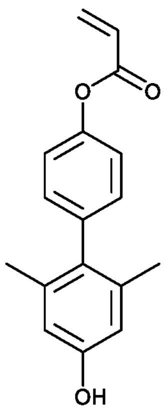 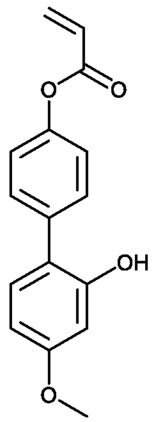 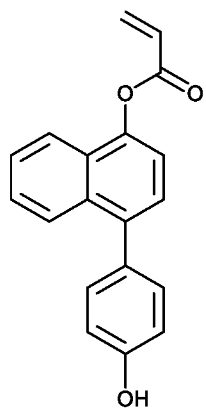 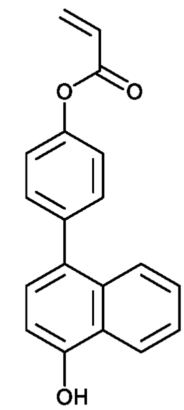

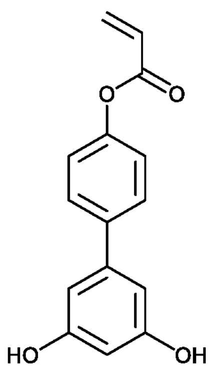 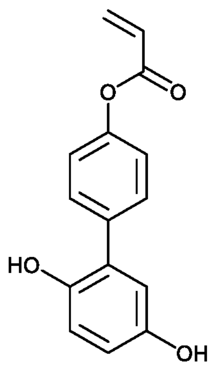 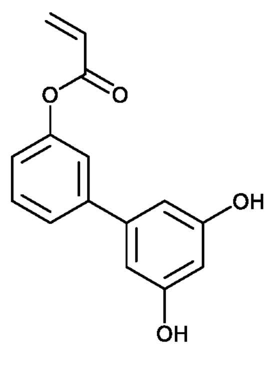

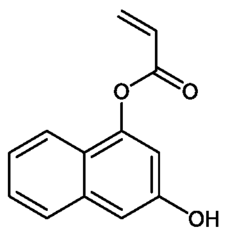
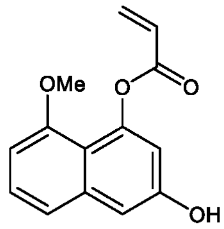
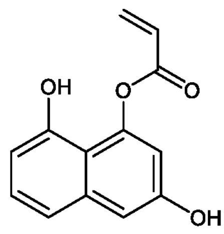

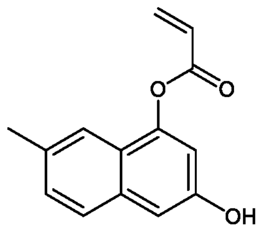
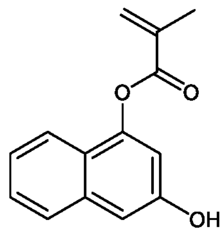
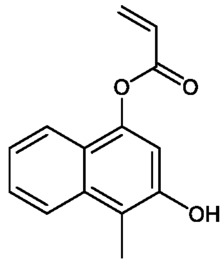

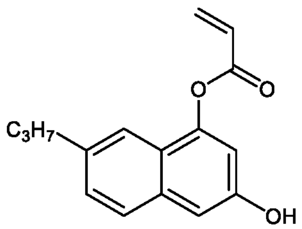
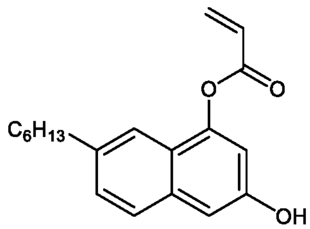
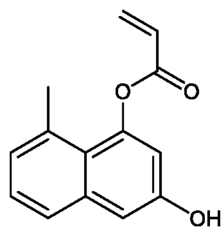

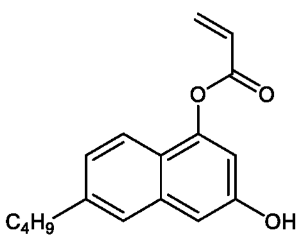
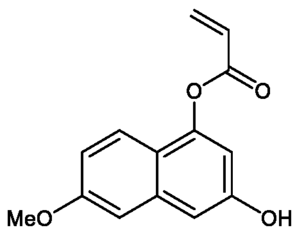
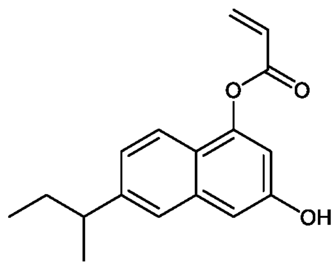

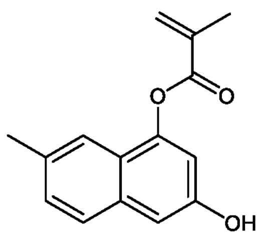
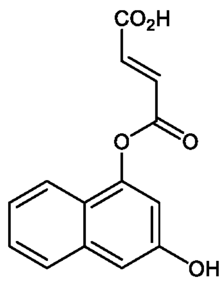

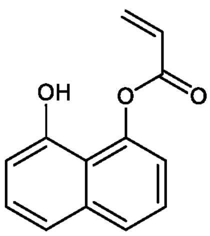
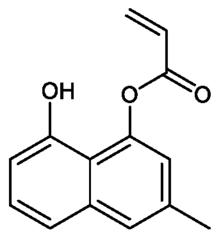
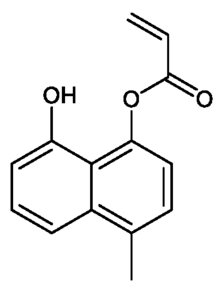

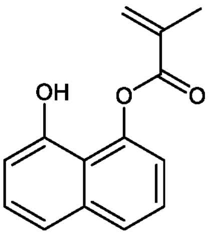

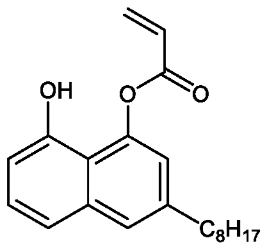

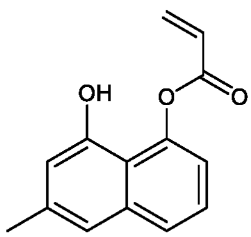

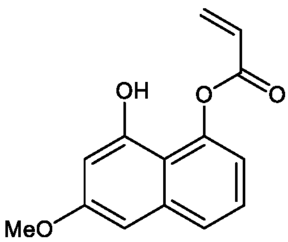

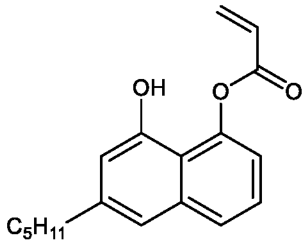

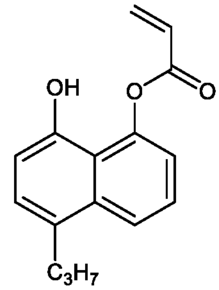

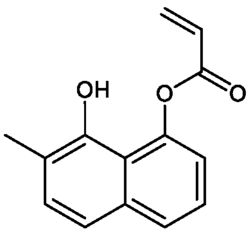

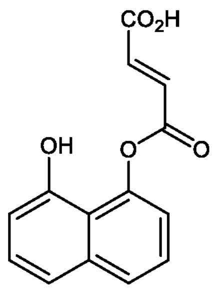

**7.** The superabsorbent polymer of claim 1,
wherein the polymerizable antibacterial monomer is included in the crosslinked polymer in an amount of 0.1 part by weight to 1 part by weight with respect to 100 parts by weight of the acrylic acid-based monomer.

**8.** The superabsorbent polymer of claim 1,
wherein the superabsorbent polymer exhibits an antibacterial property against at least one of Gram-negative bacteria and Gram-positive bacteria.

**9.** The superabsorbent polymer of claim 8,

wherein the Gram-negative bacteria is *Proteus mirabilis* or *Escherichia coli,* and
the Gram-positive bacteria is *Enterococcus faecalis.*

**10.** The superabsorbent polymer of claim 1,
wherein the superabsorbent polymer exhibits a 30-min centrifuge retention capacity (CRC) of 20 g/g to 45 g/g for physiological saline (0.9 wt% aqueous sodium chloride solution), as measured according to the EDANA method WSP 241.3.

**11.** A method of preparing a superabsorbent polymer, the method comprising the steps of:

preparing a monomer composition by mixing an acrylic acid-based monomer including acidic groups, a polymerizable antibacterial monomer represented by Formula 1, a crosslinking agent, and a polymerization initiator (step 1);
forming a water-containing gel polymer by polymerizing the monomer composition (step 2); and
drying, pulverizing, and classifying the water-containing gel polymer (step 3):

[Formula 1]

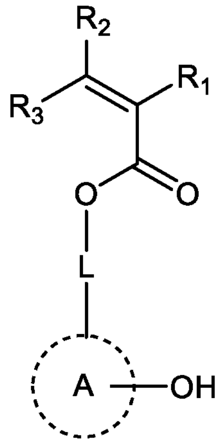

in Formula 1,
$R_1$ to $R_3$ are each independently hydrogen, alkyl having 1 to 4 carbon atoms, or a carboxyl group (COOH),
L is a single bond, or arylene having 6 to 60 carbon atoms, and
A is an aromatic ring having 6 to 60 carbon atoms,

wherein the arylene and the aromatic ring are each independently unsubstituted or substituted with one or more substituents selected from the group consisting of hydroxy, alkyl having 1 to 10 carbon atoms, and alkoxy having 1 to 10 carbon atoms.

**12.** The method of claim 11,
wherein the polymerizable antibacterial monomer is used in an amount of 0.1 part by weight to 1 part by weight with respect to 100 parts by weight of the acrylic acid-based monomer.

**13.** An article comprising the superabsorbent polymer of any one of claims 1 to 10.

**14.** The article of claim 13,
wherein the article is one or more selected from the group consisting of absorbent articles, hygiene products, water retaining soil products, water stop materials for the civil engineering and construction, sheets for raising seedling, fresh-keeping agents, materials for poultice, electrical insulators, oral care products, dental products, cosmetic products, and skin products.

【FIG. 1】

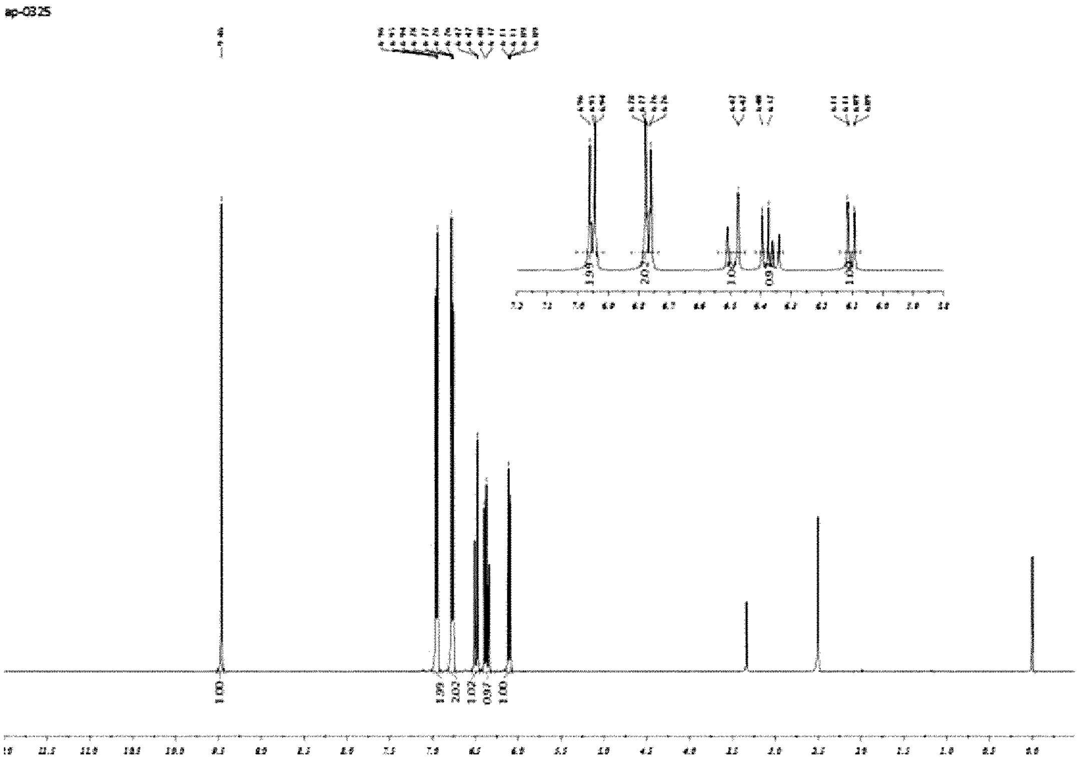

**INTERNATIONAL SEARCH REPORT**

International application No.
**PCT/KR2022/012381**

**A. CLASSIFICATION OF SUBJECT MATTER**

**C08F 220/06**(2006.01)i; **C08F 220/30**(2006.01)i

According to International Patent Classification (IPC) or to both national classification and IPC

**B. FIELDS SEARCHED**

Minimum documentation searched (classification system followed by classification symbols)

C08F 220/06(2006.01); A01N 57/34(2006.01); A01N 61/00(2006.01); C08F 2/10(2006.01); C08F 216/14(2006.01); C08F 220/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 흡수제(absorbent, SAP), 아크릴레이트(acrylate), 항균성(anti-microbial, anti-bacterial), 가교(crosslink), 제품(article)

**C. DOCUMENTS CONSIDERED TO BE RELEVANT**

| Category* | Citation of document, with indication, where appropriate, of the relevant passages | Relevant to claim No. |
|---|---|---|
| Y | JP 08-092020 A (NIPPON CHEM. IND. CO., LTD.) 09 April 1996 (1996-04-09)<br>See claims 1-4; and paragraphs [0027], [0028], [0031], [0036] and [0037]. | 1-14 |
| Y | PARK, E.-S. et al. Antimicrobial activity of phenol and benzoic acid derivatives. International biodeterioration & biodegradation. 2001, vol. 47, no. 4, pp. 209-214.<br>See abstract; figure 1; and tables 1-3. | 1-14 |
| Y | LI, X. et al. Synthesis and water absorbency of polyampholytic hydrogels with antibacterial activity. Journal of applied polymer science. 2009, vol. 112, no. 1, pp. 439-446.<br>See abstract; and pages 440 and 441. | 1-14 |
| Y | CN 1670047 A (ZHONGKAI AGROTECHNICAL COLLEGE) 21 September 2005 (2005-09-21)<br>See claims 1-9. | 1-14 |
| A | CN 108239215 A (WANHUA CHEMICAL GROUP CO., LTD.) 03 July 2018 (2018-07-03)<br>See claims 1-15; and paragraphs [0029] and [0045]-[0048]. | 1-14 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 November 2022** | **28 November 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**
**Information on patent family members**

International application No.
**PCT/KR2022/012381**

| Patent document cited in search report | Publication date (day/month/year) | Patent family member(s) | Publication date (day/month/year) |
| --- | --- | --- | --- |
| JP 08-092020 A | 09 April 1996 | JP 3625304 B2 | 02 March 2005 |
| CN 1670047 A | 21 September 2005 | CN 1296397 C | 24 January 2007 |
| CN 108239215 A | 03 July 2018 | WO 2018-120056 A1 | 05 July 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210109663
- KR 1020220102205


### Non-patent literature cited in the description


- **REINHOLD SCHWALM.** UV Coatings: Basics, Recent Developments and New Application. Elsevier, 2007, 115
- **ODIAN.** Principle of Polymerization. Wiley, 1981, 203